(19) Europäisches Patentamt
European Patent Office
Office européen des brevets

(11) EP 0 980 535 B1

(12) EUROPEAN PATENT SPECIFICATION

(45) Date of publication and mention of the grant of the patent:
**25.09.2002 Bulletin 2002/39**

(21) Application number: **98920210.6**

(22) Date of filing: **01.05.1998**

(51) Int Cl.[7]: **C07D 311/92**, C09K 9/02

(86) International application number:
**PCT/US98/09073**

(87) International publication number:
**WO 98/050807 (12.11.1998 Gazette 1998/45)**

(54) **PHOTOCHROMIC COMPOSITIONS, PHOTOCHROMIC COMPOUNDS (CO)POLYMER MATRICES**

PHOTOCHROME ZUSAMMENSETZUNGEN, PHOTOCHROME VERBINDUNGEN (KO)POLYMER MATRIZEN

COMPOSITIONS PHOTOCHROMES, MATRICES (CO)POLYMERES A COMPOSES PHOTOCHROMES

(84) Designated Contracting States:
**BE DE ES FR GB IE IT NL**

(30) Priority: **02.05.1997 FR 9705459**

(43) Date of publication of application:
**23.02.2000 Bulletin 2000/08**

(73) Proprietor: **Corning S.A.**
**77920 Samois sur Seine (FR)**

(72) Inventors:
- **BREYNE, Olivier**
**F-94380 Borneil-sur-Marne (FR)**
- **CHAN, You-Ping**
**F-69008 Lyon (FR)**
- **HENRY, David**
**F-91150 Morigny (FR)**
- **LAFOSSE, Xavier**
**F-92160 Antony (FR)**

(74) Representative: **Le Roux, Martine et al**
**Cabinet Beau de Loménie**
**158, rue de l'Université**
**75340 Paris Cédex 07 (FR)**

(56) References cited:
**WO-A-94/22850**
**WO-A-98/04937**
**US-A- 3 627 690**
**US-A- 5 623 005**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The object of the present invention is to produce particularly efficient photochromic compositions which incorporate two photochromic compounds within its matrix. The invention involves using the two photochromic compounds jointly. The invention is based on a double selection insofar as the two photochromic compounds were selected on the one hand for their intrinsic properties and on the other, for their mutual compatibility and their complementarity (in respect of the tint obtained).

The present invention finally relates to (co)polymer matrices which incorporate one or the other, advantageously both photochromic compounds as well as finished products - glazings, optical device, ophthalmic or solar article etc., constituted wholly or in part of such matrices.

The photochromic compounds are capable of changing color under the influence of a poly- or mono-chromatic light (UV for example) and of returning to their initial color when the luminous irradiation ceases, or under the influence of temperature and/or poly- or mono-chromatic light different from the first.

The photochromic compounds find applications in various fields, for example, for the manufacture of ophthalmic lenses, contact lenses, solar protection glasses, filters, camera optics or photographic apparatus optics or other optical devices and observation devices, glazings, decorative objects, bill elements or even for information storage by optical inscription (coding).

In the field of ophthalmic optics, and in particular the spectacles trade, a photochromic lens which comprises one or more photochromic compounds must have:

- a high transmission in the absence of ultraviolets,
- a low transmission (high colorability) under solar irradiation,
- adapted coloration and discoloration kinetics,
- a tint acceptable to the consumer (gray or brown preferably) with a maintenance of the chosen tint during the coloration and the discoloration of the lens,
- a maintenance of the performances, the properties, within a temperature range of 0-40°C,
- a significant durability, since these objectives sought are sophisticated corrective lenses and therefore expensive.

These lens characteristics are in fact determined by the active photochromic compounds which they contain; compounds which must furthermore be perfectly compatible with the organic or inorganic support which constitutes the lens.

Moreover, it is to be noted that obtaining a gray or brown tint - tints acceptable to the consumer - necessitates the use in practice of at least two photochromes of different colors, i. e. having distinct maximal absorption wavelengths in the visible. This association further imposes other requirements of the photochromic compounds. In particular, the coloration and discoloration kinetics of the (two or more) associated active photochromic compounds must be essentially identical. It goes without saying for their stability with time and also for their compatibility with a plastic or inorganic support.

Amongst the numerous photochromic compounds described in the prior art, benzopyrans and naphthopyrans may be cited which are described in patents US-A-3,567,605, US-A- 3,627,690, US-A- 4,818,096, US-A- 4,826,977, US-A-5,200,116, US-A- 5,238,981, US-A- 5,458,814, WO 96/04576 and in the Research Disclosure No. 36144, which are of the formula below :

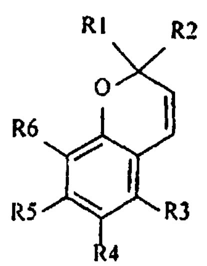

These compounds claim to satisfy the specifications defined above. In reality, if these compounds really do have one or more of the basic properties sought, such as a high transmission in the absence of ultraviolets and a high colorability under solar irradiation, none of them have the complete combination of the properties sought which are necessary for the production of satisfactory articles which may be manufactured industrially.

The Applicant, confronted with the specifications proposes a novel solution which is based on a double selection.

It is in fact to the credit of the Applicant to have selected on the one hand, a photochromic compound (compound (I) of the invention which exhibits a violet color) amongst the compounds described in its patent application WO-A-98 04937, and on the other hand, a photochromic compound (compound (II) of the invention which exhibits a yellow color) amongst the compounds described in the application WO-A-94 22850; compounds ((I) and (II)). both efficient *per se*, and whose association, in addition, has revealed to be particularly interesting insofar as the two compounds, which are perfectly compatible, are complementary in exhibiting a gray tint (acceptable to the consumer).

Thus, according to its first object, the invention relates to a photochromic composition incorporating

+ 2-(p-dimethylaminophenyl)-2-(p-methoxyphenyl)-5-methyl-7,9-dimethoxy -[2H]-naphtho[1,2-b]pyran (compound (I)),

  and

+ 3-(p-methoxyphenyl)-3-phenyl-6-morpholino-3H-naphtho-[2,1-b]pyran (compound (II)).

The photochromic composition advantageously contains compounds (I) and (II) in a compound (I) / compound (II) weight ratio between 7 and 10 (more advantageously still between 8 and 9). The best results of the exhibition of the gray tint are within the context of this advantageous variant.

According to its first object, the present invention therefore relates to the joint use of 2-(p-dimethylaminophenyl)-2-(p-methoxyphenyl)-5-methyl-7,9-dimethoxy -[2H]-naphtho[1,2-b]pyran (compound (I)) and 3-(p-methoxyphenyl)-3-phenyl-6-morpholino-3H-naphtho-[2.1-b]pyran (compound (II)) as photochromic agents; the two photochromic agents being advantageously used in a compound (I) / compound (II) weight ratio between 7 and 10 (more advantageously still between 8 and 9). Such a joint use is generally made within a polymeric matrix to which it is desired to confer photochromic properties

Such a joint use has revealed to be particularly interesting insofar as the photochromic compounds (compound (I) and compound (II)) each have interesting photochromic properties which are compatible (notably from a kinetics and thermal point of view) and complementary with the color (see Examples below).

Compounds (I) and (II) of the photochromic compositions of the invention are now described below in detail. The formula developed for each one of them as well as a means of synthesis of same appear in the Example section of the present text. These compounds are presently claimed.

Compound (I) of the invention consists of 2-(p-dimethylaminophenyl)-2-(p-methoxyphenyl)-5-methyl-7,9-dimethoxy-[2H]-naphtho[1,2-b]pyran.

It is of the general formula of the photochromic naphthopyrans described by the Applicant in its application WO-A-98 04937 claiming a priority of the 25 July 1996 ; general formula :

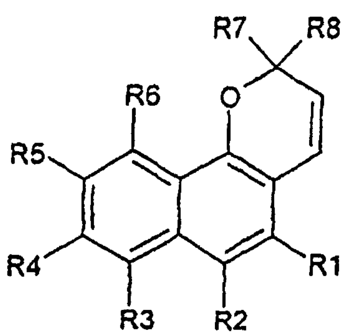

in which at least two of the substituents $R_3$ to $R_6$ (advantageously $R_3$ and $R_5$) are C1 to C6 alkoxy groups(advantageously methoxy groups). In application WO-A-98 04937, $R_7$ and $R_8$ are described in a very broad manner, notably as aromatic or polyaromatic groups optionally substituted with at least one C1 to C5 alkoxy, C1 to C5 alkyl, C2 to C12 amine, C6 to C12 aryl, or $CF_3$ group. In said application WO-A-98 04937, a conventional synthetic route to naphthopyrans is further described : condensation of an appropriately substituted 1-naphthol derivative (substituents $R_1$ to $R_6$) and a propargylic alcohol derivative (substituents $R_7$ and $R_8$).

Within the context of the present invention, the Applicant has selected compound (I) - a violet molecule - of the general formula above in which:

. $R_1 = CH_3$

. $R_2 = R_4 = R_6 = H$

. $R_3 = R_5 = O\ CH_3$

. $R_7$= (para) 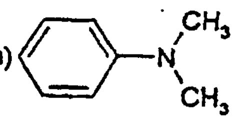

. $R_8$ = (para) 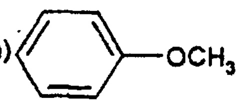

Surprisingly, the compound has very interesting photochromic properties which are superior to those of its homologues; and has notably:

- a weak initial coloration,
- a strong colorability with two absorption bands in the visible (see the Table below: λmax 1, λmax 2),
- discoloration kinetics adapted (to the application sought),
- a low thermal dependence.

The Applicant has notably evaluated the properties in an original matrix which is specified further on in the present text. The matrix is based on a short-chain (meth)acrylate difunctional monomer and a long-chain alkenic difunctional monomer. Used at 0.05% by weight within the matrix (conditioned under 2 mm thickness), the compound has led to the results shown in the Table below.

| Compound | λmax 1 | λmax 2 | T0* at 489 nm | T0* at 588 nm | DOinduced** at 489 nm | DOinduced** at 588 nm | $t_{1/2}$*** |
|---|---|---|---|---|---|---|---|
| I | 489 nm | 588 nm | 85 % | 86 % | 0.58 | 0.94 | 127 s |

* T0 : transmission before exposure.

** DOinduced = $DO_x = DO_0$ wherein $DO_0$ is the optical density before exposure and $DO_x$ the optical density after exposure under a xenon lamp (40.000 lux).

*** $t_{1/2}$ = half-time of fading (expressed in seconds). This parameter characterizes the kinetics of return to the initial state. After 15 minutes' exposure under the above conditions ($DO_{15}$), the exposure is cut off and the time necessary for a return to $\frac{DO_{15} - DO_0}{2}$ is timed; this is $t_{1/2}$.

Upon considering the results, the person skilled in the art grasps straight away the beneficial advantages of compound (I) selected amongst the numerous naphthopyrans of the application WO-A-98 04937. Compound (I) must nonetheless be associated with a complementary yellow photochrome in order to provide a gray tint.

Compound (II) of the invention consists of 3-(p-methoxyphenyl)-3-phenyl-6-morpholino-3H-naphtho-[2,1-b] pyran. It is of the general formula of the photochromic naphthopyrans described in the application WO-A-94 22850 ; general formula :

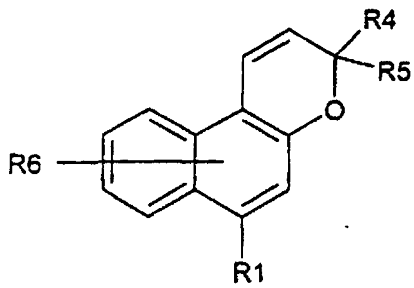

in which :

- $R_1$ represents an amine group (advantageously morpholino or piperidino);
- $R_1$ and $R_5$ independently represent an alkyl. an alkenyl, a heterocyclic or carbocyclic group (advantageously, a phenyl, p-methoxyphenyl or trifluoromethylphenyl) or are bound in order to represent a ring or a hetrocyclic ring ;
- $R_6$ represents a hydrogen or a substituent.

Naphthopyrans characteristically have a substituted amino group in position 6 (substituent $R_1$). They are obtained by a synthetic route analogous to that indicated above with reference to the compounds described in the application WO-A-98 04937: condensation of an appropriately substituted 2-naphthol derivative (substituents $R_1$ and $R_6$) and a propargylic alcohol derivative (substituents $R_4$ and $R_5$).

Within the context of the present invention, the Applicant has selected compound (II) - a yellow molecule - of the above formula in which :

. $R_1$ = morpholino
. $R_4$ = phenyl
. $R_5$ = p-methoxyphenyl
. $R_6$ = H.

Compound (II) is superior to its homologues insofar as a compromise in terms :

- of weak initial coloration,
- of high colorability,
- of discoloration kinetics, and
- of thermal dependence

is obtained along with it.

This affirmation is corroborated by the results given in the Table below. Compounds C1, C2, C3, other naphthopyrans according to the application WO-A-94 22850, are identified by their developed formula in the Example part of the text. All compounds (II), C1, C2, C3 were tested under the same conditions as those indicated above with reference to compound (I).

| Compound | λmax (nm) | T0 (%) | DO induced | $t_{1/2}$ (s) |
|---|---|---|---|---|
| II | 439 | 79 | 1.65 | 88 |
| C1 | 456 | 74 | 1.37 | 52 |
| C2 | 430 | 68 | 1.60 | 150 |
| C3 | 430 | 79 | 1.77 | 164 |

Compound (II) furthermore reveals to constitute a partner of choice for compound (I), in a way as to express a tint acceptable to the consumer, i. e. gray, under favorable conditions (of initial color, of interesting colorability, of adapted discoloration and of acceptable thermal dependence).

Compound (II) has in fact the specifications which determine the profile of an efficient partner of compound (I). The Applicant, after analyzing the spectral curve of compound (I) had established such specifications. It is summarized in the Table below:

| λmax (nm) | T0 (%) | DO induced | $t_{1/2}$ (s) |
|---|---|---|---|
| 435 - 450 | > 77 | > 1.50 | < 127 |

The Applicant has therefore, within the context of the present invention, selected two compatible efficient photochromic compounds which are claimed either individually or in a mixture.

It is hereby indicated that in general, the photochromic composition of the invention only contains the two compounds in a weight ratio which is optimized in order to obtain a gray tint sought-after. However, it is in no way excluded from the context of the present invention that the composition contains at least one other obviously compatible, photochromic compound.

The compounds of the invention and their mixtures ((I), (II), (I) + (II)) may be dispersed uniformly in the mass or on the surface of a polymer matrix. The most interesting applications of the compounds of the invention are in fact those in which the photochrome(s) is (are) uniformly dispersed within or on the surface of a polymer, a copolymer or a mixture of polymers. The (co)polymer matrix which comprises the photochromes of the invention (compound (I), compound (II)) and advantageously the mixture of photochromes of the invention constitutes another object of the present invention.

The implementation methods envisagable for obtaining such a matrix are very varied. Amongst those known to the person skilled in the art, diffusion in the (co)polymer from suspension or solution of photochrome(s), in a silicone oil, in an aliphatic or aromatic hydrocarbon, in a glycol, or from another polymer matrix, may be cited for example. The diffusion is commonly effected at a temperature of 50 to 200°C for a period of time of 15 minutes to several hours, according to the nature of the polymer matrix. Another implementation technique consists in mixing the photochromc (s) in a formulation of polymerizable materials, in depositing this mixture on a surface or in a mold and in then carrying out the copolymerization. These and other implementation techniques are described in the article by Crano *et al*. "Spiroxazines and their use in photochromic lenses" published in Applied Photochromic Polymer Systems, Ed. Blackie and Son Ltd - 1992.

The following products ((co)polymers) may be mentioned as examples of preferred polymer materials for optical applications of the photochromic compounds of the invention, taken alone or in a mixture:

a) optionally halogenated alkyl, cycloalkyl, aryl or aralkyl mono-, di-, tri- or tetraacrylate or mono-, di-, tri- or tetramethacrylate or having at least one ether and/or ester and/or carbonate and/or carbamate and/or thiocarbamate and/or urea and/or amide group,
b) polystyrene, polyether, polyester, polycarbonate, polycarbamate, polyepoxy, polyurea, polyurethane, polythiourethane, polysiloxane, polyacrylonitrile, polyamide, aliphatic or aromatic polyester, vinylic polymer, cellulose acetate, cellulose triacetate, cellulose acetate-propionate or polyvinylbutyral,
c) copolymers of two or more types of monomer or mixtures of polymers mentioned above,
d) copolymers obtained by radical polymerization of a composition comprising a mixture of at least one or more difunctional monomers of type (a) and one or more difunctional monomers of type (b):

* the difunctional monomer(s) of type (a) having one or the other of the formulae (A) and (A') below :

+ formula (A) :

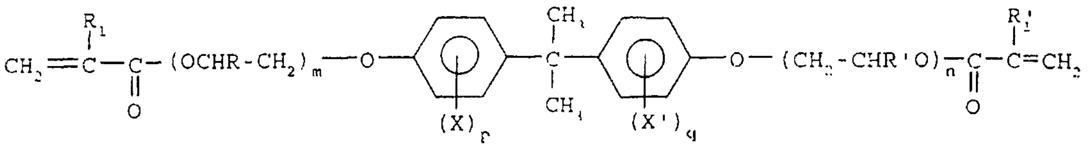

in which :

- $R_1$, $R'_1$, R and R', identical or different, independently are a hydrogen or a methyl group ;
- m and n arc, independently, integers between 0 and 4 inclusive ; and arc advantageously independently equal to 1 or 2 ;
- X and X', identical or different, are a halogen and preferably represent chlorine and/or bromine;
- p and q are, independently, integers between 0 and 4 inclusive ;

+ formula (A') :

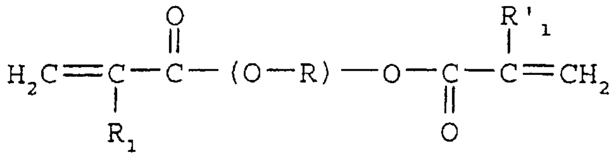

in which :

- $R_1$ and $R'_1$, identical or different, independently are a hydrogen or a methyl group ;
- R is a linear or branched alkyl radical having from 2 to 8 carbon atoms, a cycloalkyl radical having from 3 to 6 carbon atoms, an ether radical of formula (R'-O-R") in which R' and R", identical or different, independently arc a linear or branched alkyl radical having from 2 to 4 carbon atoms;

* the difunctional monomer(s) of type (b) - long chain alkenic difunctional oligomer - being of one or the other of formulae (B), (B') and (B") below:

+ formula (B) :

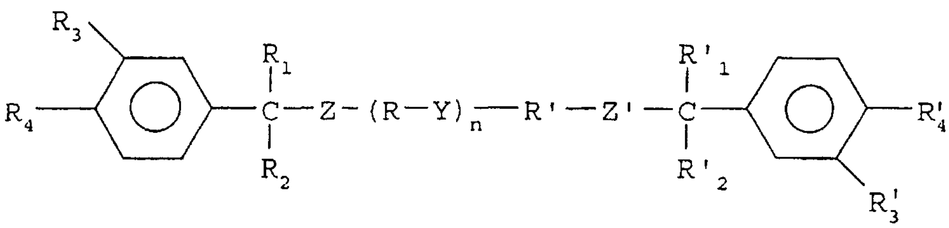

in which :

- $R_1$, $R'_1$, $R_2$ and $R'_2$, identical or different, independently are hydrogen or a linear or branched alkyl radical, advantageously linear, having from 1 to 4 carbon atoms ; and correspond particularly advantageously to a methyl group ;
- $R_3$ and $R_4$, different, are independently one hydrogen and the other an alkenyl radical having from 2 to 6 carbon atoms, advantageously from 2 to 4 carbon atoms and particularly advantageously an isopropenyl radical ;
- $R'_3$ and $R'_4$, different, arc independently one hydrogen and the other an alkenyl radical having from 2 to 6 carbon atoms, advantageously from 2 to 4 carbon atoms and particularly advantageously an isopropenyl radical ;
- Z represents a carbamalc function (-NH-CO-O-), a thiocarbamate function (-NH-CO-S-) or a urea function (-NH-CO-NH-) ;
- Z', independent from Z and advantageously respectively with respect to Z, represents a carbamate function (-O-CO-NH-), a thiocarbamate function (-S-CO-NH-) or a urea function (-NH-CO-NH-);
- R' represents a linear or branched alkyl radical having from 2 to 4 carbon atoms ;
- R, identical or different when $n \geq 2$, is a linear or branched alkyl radical having from 2 to 4 carbon atoms ;
- Y, identical or different when $n \geq 2$, is oxygen or sulfur ;
- n is an integer defined in such a way that the total number of carbon atoms contained in the long chain situated between the two motifs Z and Z' is at least equal to 18 and is advantageously between 18 and 112 inclusive ;

+ formula (B') :

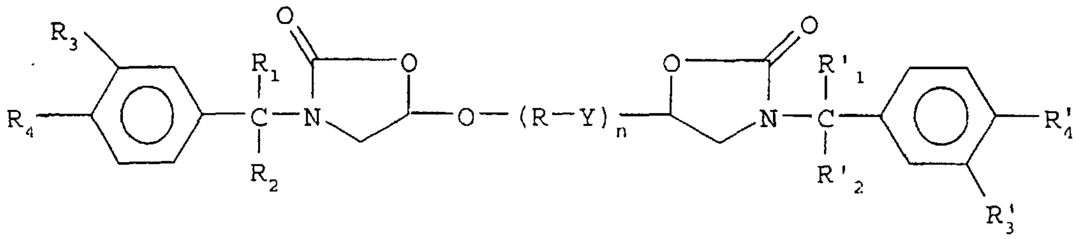

in which :

- $R_1$, $R_2$, $R_3$, $R_4$, $R'_1$, $R'_2$, $R'_3$, $R'_4$, R and Y are such as defined above with reference to formula (B);
- n is an integer defined in such a way that the total number of carbon atoms contained in the long chain of the motif $(R\text{-}Y)_n$ is at least equal to 22 and is advantageously between 22 and 104 inclusive;

+ formula (B")

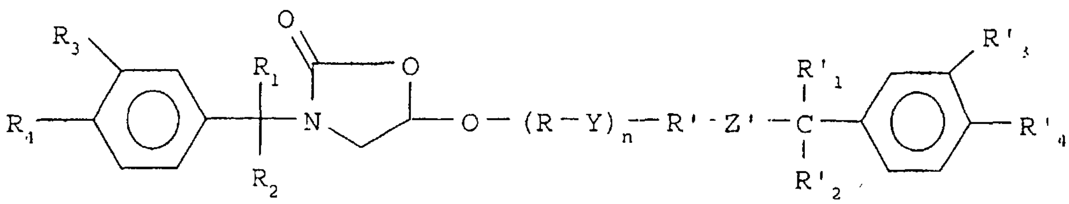

in which :

- $R_1$, $R_2$, $R_3$, $R_4$, $R'_1$, $R'_2$, $R'_3$, $R'_4$, R, R' and Y are such as defined above with reference to formula (B);
- Z' is a carbamate function (-O-CO-NH-) or Z' is a thiocarbamate function (-S-CO-NH-), advantageously Z' is a carbamate function;
- n is an integer defined in such a way that the total number of carbon atoms contained in the long chain of the motif $(R\text{-}Y)_n\text{-}R'$ is at least equal to 22 and is advantageously between 22 and 104 inclusive.

It is most particularly recommended to bring in the compounds of the invention (advantageously in a mixture) in a matrix of type d) above. Such a matrix exhibits good optical properties. Within it, the photochromic compounds of the invention rapidly and at best express their excellent photochromic properties. Such a matrix is obtained by radical polymerization of at least one long-chain alkenic difunctional monomer with at least one short-chain (meth)acrylic difunctional monomer. The resulting matrix has then a nanophasic structure which provides it with interesting properties. In fact, the short-chain (meth)acrylic difunctional monomer(s) (of type (a) and of formula (A), (A')) bring about rigidity, this rigidity being modulated by the presence of the long-chain alkenic difunctional monomer(s) (of type(b) and of formula (B), (B'), (B")) which, in a surprising way, also enable providing the composition with excellent photochromic properties. Thus. the difference in functionality of the monomers of type (a) and (b) advantageously retards the gelling of the resulting polymerizable composition. Such a matrix is claimed by the Applicant in a parallel application. The nature and the advantageous variants of it are specified below.

It is possible for the polymerisable composition from which it is obtained to contain, in addition to the difunctional monomers of type (a) and (b):

(c) at least one aromatic monovinylic monomer of formula (C) :

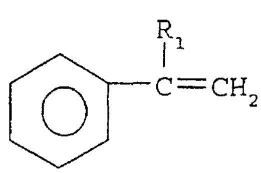

in which $R_1$ = H or $CH_3$ ; the monovinylic monomer advantageously consisting of styrene ;

and/or

(d) at least one aromatic divinylic monomer of formula (D):

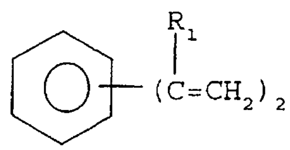

in which $R_1$ = H or $CH_3$ ; the divinylic monomer advantageously consisting of divinylbenzene;

and/or

(e) at least one (meth)acrylic monomer of formula (E) :

$$CH_2 = C(R) -COOR'$$

in which R = H or $CH_3$ and R' is a linear or branched alkyl radical having from 4 to 16 carbon atoms, an optionally substituted (generally by a $C_1$-$C_6$ alkyl group) methylphenyl or methylphenoxy radical or a polyoxyethoxyl group of formula $-(CH_2-CH_2-O)_nR''$ in which n is an integer between 1 and 10 and R'' = $CH_3$ or $C_2H_5$ ; the (meth)acrylic monomer advantageously consisting of ethylhexylmethacrylate ;

and/or

(f) diallylphthalate.

The polymerizable composition generally contains an effective amount of at least one radical polymerization initiator as well, and an effective amount of at least one polymerization modifier, the polymerization modifier being preferably a chain transfer agent.

The natures and the amounts of each one of the intervening compounds or those which can intervene in the polymerizable compositions of the invention, and notably in the preferred compositions of the invention, which generate the matrices of type (d) by copolymerization within which matrices the photochromic compounds of the invention (compound (I) and/or (II)) advantageously intervene shall now be examined in greater detail.

The monomers of type (a) of formula (A) and (A') constitute the short-chain difunctional (meth)acrylate monomers (i.e. diacrylates, dimethacrylates or mixed: acrylates-methacrylates) of the polymerizable composition(s) of the invention. The monomers may or may not have a pronounced symmetry (R/R', $R_1$/$R'_1$, X/X'). They enable conferring the rigidity, and therefore the mechanical properties, to the polymer (to the resin or matrix) obtained from the polymerizable composition.

The monomers of type (a) may or may not all be of the same formula (A) or (A') ... Thus the polymerisable compositions, precursors of the preferred matrices contain :

- either monomers of a same formula (A) (at least one);
- or monomers of a same formula (A') (at least one);
- or mixtures (non mixed) of monomers of different formulae (A) ;
- or mixtures (non mixed) of monomers of different formulae (A') ;
- or mixtures (mixed) of monomers of formula(e) (A) and of formula(e) (A').

According to a preferred variant of the invention, one or more symmetrical monomers of type (a) are used. As contemplated by the present invention, monomers of type (a), of formula (A) or (A') in which the $R_1$ and $R'_1$ groups are identical, the same as R and R' groups as well as the X and X' substituents for the compounds of formula (A) are deemed to be symmetrical.

The symmetrical monomers of type (a) of formula (A) are known and arc available commercially or are easily accessible to the person skilled in the art. In fact, it may be noted that the monomers which do not have a halogen on the aromatic rings correspond to the first monomers of formula (I) in the sense of the WO-A-92/05209 document. The monomers of type (a) of formula (A) having halogen(s) on the aromatic ring(s) will be easily obtained by the person skilled in the art by using derivatives appropriately substituted on the aromatic ring(s). Within the context of the invention, the monomers of formula (A), in which R and R', identical, are hydrogen or a methyl group, $R_1$ and $R'_1$ are a methyl group, m and n are independently equal to 1 or 2, and p = q = 0, are preferred. A particularly advantageous variant corresponds to the monomer of formula (A) of the above type with, in addition, R = R' = H and m = n = 2. The monomer

is marketed by Akzo Nobel (NL) under the commercial designation DIACRYL 121. The synthesis of the dissymmetrical monomers of formula (A) are of no particular problem to the person skilled in the art,

The monomers (a) of formula (A') are also well-known and result from the conventional reaction of an aliphatic diol or of a short-chain alkyleneglycol (with a maximum of 8 carbon atoms in the chain) with at least one type of (meth) acrylic derivative depending on whether it is desired to obtain monomers of formula (A') which arc symmetrical or dissymmetrical at their ends.

These monomers of type (a) intervene generally in the composition to be polymerized at a rate of 40 to 99 parts by weight for 100 parts by weight of the mixture of monomers of type (a) and (b). If they intervene in a smaller amount, the polymerizable composition has a tendency to retract during its polymerization inducing a premature turn-out which, in turn, is responsible for a deterioration of the optical properties of the final resin.

The monomers (b) of formula (B), (B') and (B") constitute the long-chain difunctional alkenic monomers of the polymerizable composition of the invention. The monomers have or have not a more or less pronounced symmetry ($R_1/R'_1$, $R_2/R'_2$, $R_3/R'_3$, $R_4/R'_4$, Z/Z').

These monomers of type (b) may or may not all have the same formula (B), (B') or (B") ... Thus, the invention comprises as well the polymerizable compositions which contain:

- either monomers of a same formula (B) (at least one);
- or monomers of a same formula (B') (at least one);
- or monomers of a same formula (B") (at least one);
- or mixtures (non mixed) of monomers of different formulae (B) ;
- or mixtures (non mixed) of monomers of different formulae (B') ;
- or mixtures (non mixed) of monomers of different formulae (B") ;
- or mixtures (mixed, binary or ternary) of monomers selected from the monomers of formula(e) (B), of formula(e) (B') and of formula(e) (B").

The presence of monomer(s) of type (b) in the polymerizable composition of the invention allows a softening of the polymer network by loosening the network without lowering for as much the degree of cross-linking of the polymer. This allows conferring interesting mechanical properties to the material at high temperature, characterized notably by a high elasticity modulus value at the rubbery "plateau" of the polymer.

The monomers of type (b) which are <u>long-chain</u> alkenic difunctional oligomers, the chain being a polyoxyalkylene or polymercaptoalkylene chain, even a mixed chain, are obtained according to the conventional conditions of organic synthesis by the reaction:

- between one or several derivatives having a functionality of the alkcnylisocyanate type, of formula I and/or II:

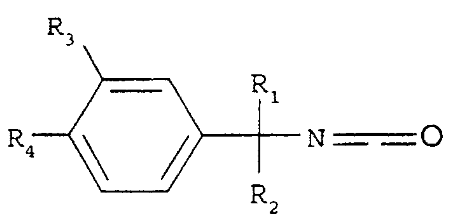

formula I

and/or

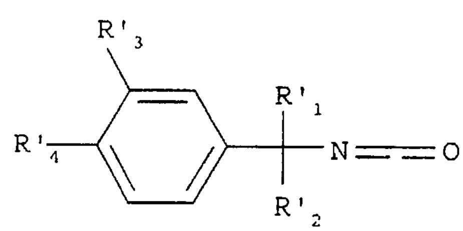

formula II

  in which $R_1$, $R_2$, $R_3$, $R_4$, $R'_1$, $R'_2$, $R'_3$ and $R'_4$ are such as defined above. According to a preferred variant of the invention, the monomers of type (b) used are symmetrical at their ends. In order to do this, a single type of alkenylisocyanate derivative (thus formulae I and II arc identical) is used. In a particularly advantageous way, a vinylisocyanate derivative is used in which $R_1=R_2=CH_3$ (or $R'_1=R'_2=CH_3$), $R_3$ (or $R'_3$) is an isopropenyl radical and $R_4$ (or $R'_4$) is hydrogen, thus corresponding to the 3-isopropenyl-α,α-dimethylbenzylisocyanate (of general designation m-TMI® defined above). The oligomers (b) obtained from the derivatives are preferred;
- and a compound which intrinsically has a long chain, the compound being:

  * either a compound which is symmetrical about its terminal functions which correspond:

    + to a diol of formula $HO-(R-Y)_n-R'-OH$;
    + or to a dithiol of formula $HS-(R-Y)_n-R'-SH$:
    + or to a diamine of formula $H_2N-(R-Y)_n-R'-NH_2$; which allows obtaining the intrinsically symmetrical oligomers of formula (B) (intrinsically symmetrical means monomers of formula (B) in which the Z and Z' groups are functions of identical nature);

+ or to a biepoxy of formula

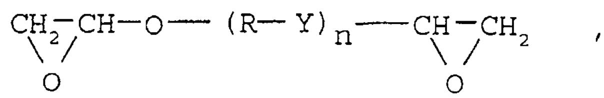

the reaction thus leading to the synthesis of the oligomers of formula (B');

* or a compound which is dissymmetrical about its terminal functions :

+ it being possible for the functions to be an alcohol, thiol or amine function; all combinations being possible: these compounds enabling obtaining other intrinsically dissymmetric difunctional oligomers of formula (B) (intrinsically dissymmetrical means monomers of formula (B) in which the Z and Z' groups are functions of different nature);
+ the functions respectively being an epoxy function and an alcohol function or an epoxy function and a thiol function, the compounds then being of formula

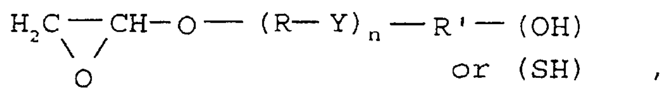

the reaction thus leading to the synthesis of the oligomers of formula (B").

In every case, R, R' ,Y and n are such as defined above; preferentially, Y is oxygen (the long chain then being a polyoxyalkylene chain).

The molecular mass of the long polyoxyalkylene and/or polymercaptoalkylene chain which correspond to the $(R-Y)_n-R'$ or $(R-Y)_n$ radical in formulae (B, B', B") specified above, is generally at least equal to 500 $g.mol^{-1}$ and lower than 2000 $g.mol^{-1}$; and preferably, the molecular mass is between 600 $g.mol^{-1}$ and 900 $g.mol^{-1}$.

In a particularly advantageous way, one or several intrinsically symmetrical monomers of type (b) of formula (B) (as defined above) arc brought to intervene:

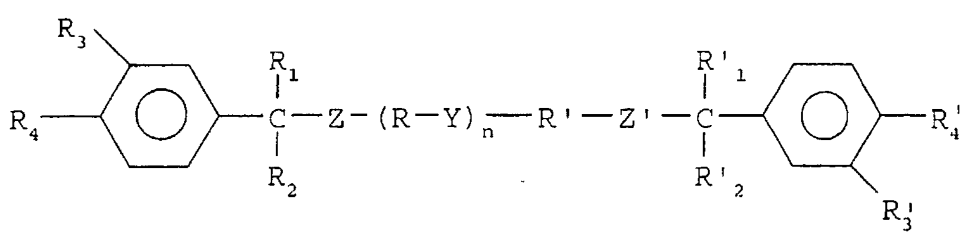

in which R, R', $R_1$, $R_2$, $R_3$, $R_4$, $R'_1$, $R'_2$, $R'_3$, $R'_4$ and Y are such as defined above (and advantageously so that the two end of the molecule are identical; i.e. : $R_1=R'_1$, $R_2=R'_2$, $R_3=R'_3$ and $R_4 = R'_4$, with even more advantageously $R_1=R'_1=R_2 = R'_2 = CH_3$ and $R_3 = R'_3$ and $R_4 = R'_4$ with one of $R_3$ and $R_4$ being hydrogen and the other being an isopropenyl group) and Y is such as defined above and advantageously consists of an oxygen (X=O) and:

(α)

- Z and Z' are carbamate functions of formula (-NH-CO-O-) and (-O-CO-NH-) respectively;
- n is an integer defined in such a way that the total number of carbon atoms contained in the long chain situated between the two motifs Z and Z', is between 18 and 112 ; and advantageously, in the case of a polyoxyalkylene chain, is between 24 and 112 and particularly advantageously between 26 and 50 in the case of a polyoxyalkylene of molecular mass between 600 and 900 $g.mol^{-1}$;

or (β)

- Z and Z' are thiocarbamatc functions of formula (-NH-CO-S-) and (-S-CO-NH-) respectively
- n is an integer defined in such a way that the total number of carbon atoms contained in the long chain situated between the two motifs Z and Z', is between 18 and 108 ; and advantageously, in the case of a polyoxyalkylene chain, is between 24 and 108 and particularly advantageously between 28 and 46 in the case of a polyoxyalkylene chain of molecular mass between 600 and 900 $g.mol^{-1}$;

or ($\gamma$)

- Z and Z' are urea functions (-NH-CO-NH-)
- n is an integer defined in such a way that the total number of carbon atoms contained in the long chain situated between the two motifs Z and Z', is between 18 and 112; and advantageously, in the case of a polyoxyalkylene chain, is between 24 and 112, and particularly advantageously between 28 and 50 in the case of a polyoxyalkylene of molecular mass between 600 and 900 $g.mol^{-1}$.

The person skilled in the art will have understood that formula (B), in the case ($\alpha$) above wherein the number of carbon atoms contained in the long chain is equal to 50, may for example be written:

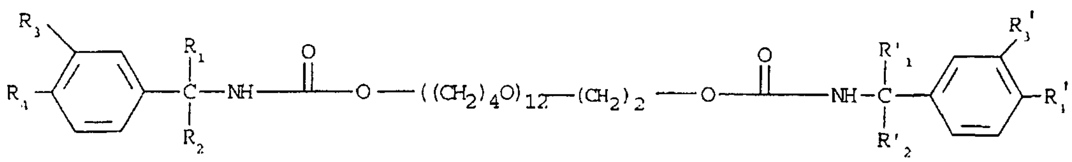

In the same way. the person skilled in the art will have understood that generally the minimal values mentioned above which define the number of carbon atoms in the long chain of motif $(R-Y)_n$-R' or $(R-Y)_n$, correspond to compounds having a polymercaptoalkylene chain (Y = S).

In a particularly advantageous manner, the monomer(s) of type (b) have a general formula (B) such as defined above in which:

- $R_1$,$R_2$, $R'_1$ and $R'_2$, identical, are methyl radicals; $R_3$ and $R'_3$ are an isopropenyl radical; $R_4$ and $R'_4$ are hydrogen and
  + either Z and Z' are urea functions (-NH-CO-NH-) and

- R' represents an ethylene or propylene group;
- n is an integer equal to 13 or 19 which defines a total number of carbon atoms between Z and Z' equal to 28 or 40 when $(R-Y)_n$ is a polyoxyethylene chain; or n is an integer equal to 10 or 14 which defines a total number of carbon atoms between Z and Z' equal to 33 or 45 when $(R-Y)_n$ is a polyoxypropylene chain; or n is an integer between the lower limit values (10 to 13) and upper limit values (14 to 19) such as defined above, when $(R-Y)_n$ is a polyoxyethylene / polyoxypropylene mixed chain;

  + or Z and Z' are carbamate functions of formulae (-NH-CO-O-) and (-O-CO-NH-) respectively, and

- R' represents an ethylene group ;
- $(R-Y)_n$ represents a long polyoxyethylene chain;
- n is an integer equal to 13 or 19 which defines the total number of carbon atoms contained in the long chain situated between the two motifs Z and Z' equal to 28 or 40.

The monomers of type (b) generally intervene in the composition to be polymerized at a rate of 1 to 60 parts by weight for 100 parts by weight of the mixture of monomers of type (a) and (b).

The polymerizable composition may furthermore contain, as already indicated, other monomers. Generally, for 100 parts by weight of the mixture of monomers of type (a) and (b), the composition can contain from 1 to 60 parts by weight (advantageously from 10 to 50 parts by weight) of at least one monomer selected from the alkenic monomers (such as those of formulae (C ) and (D) and diallylphthalate (f)), advantageously vinylic and allylic, (meth)acrylic monomers (such as those of formula (E)) and mixtures thereof. In light of the effects sought-after, when these types of monomer are added, the person skilled in the art will know to determine and optimize the intervening amounts of each

type of the monomer (in any case, the total amount of the monomer(s) which intervene in the polymerizable composition is between 1 to 60 parts by weight of the mixture of monomers of type (a) and (b)).

The vinylic monomers of formula (C ) - styrene and/or methylstyrene - intervene in combination with the monomcr(s) of type (a) in order to loosen the network. The intervention of styrene may be particularly advantageous insofar as this polymerized compound has a rather high refractive index (n = 1.595). Styrene constitutes the particularly preferred compound of this class of monomer.

The compound of formula (D) consists of divinylbenzene (DVB) or di(methylvinyl)benzene. Divinylbenzene is the particularly preferred compound of formula (D). The intervention of at least one compound of formula (D) may reveal to be advantageous in that notably the compound moderates, in a general manner, the effects of the compound (s) of formula (C ). The beneficial action of such a compound of formula (D) has been notably demonstrated on the expression of photochromic properties. With reference to divinylbenzene, insofar as this polymerized compound has a relatively high refractive index (n = 1.61), its intervention is also beneficial in that it leads to an increase in the refractive index of the polymers of the invention.

The polymerizable composition also contains advantageously at least one compound of formula (E). It is a (meth)acrylic monomer such as defined above. It may notably he butyl, pentyl, hexyl, heptyl, octyl or 2-ethylhexyl(meth) acrylate or even ethyltriglycol(meth)acrylate. 2-Ethylhexylmethacrylate (EHMA) is the preferred compound of formula (E). The presence of this type of compound has revealed to be advantageous for the turning-out of the polymerized material and for the implementation of finishing treatments of the latter.

Finally, the polymerizable composition may contain diallylphthalate which notably allows adjusting the index and/or other optical and mechanical properties.

As specified above, the intervention of the compounds of formula (C ) and/or (D) and/or (E) and/or diallylphthalate is not obligatory. It does however reveal to be generally advantageous.

The monomers of types (a), (b) and (f) and of formulae (C ), (D) and (E) are the principal constituents - insofar as they intervene or can intervene in relatively consequent amounts - of the polymerizable compositions from which copolymers or resins or matrices are generated, within which the photochromic compounds (I) and/or (II) of the invention are made to intervene. The copolymers are obtained from the monomers by a conventional radical copolymerization process. The copolymerization is generally carried out as specified above in the presence of an effective amount of at least one polymerization modifier and at least one radical polymerization initiator.

The polymerization modifier generally intervenes at a maximal rate of 5% by weight, advantageously at the rate of 0.01 to 2% by weight, with respect to the weight of monomers to be copolymerized. It is hereby noted that it is possible to do away with the presence of such a polymerization modifier in the hypothesis where the material is prepared under a reduced thickness (e < 2.0 mm). In that situation, the problem associated with the evacuation of heat arc not encountered. For the preparation of a resin of the invention having a thickness greater than 2.0 mm, the presence of a polymerization modifier in the amounts indicated above is generally opportune. It is highly advised against going over the maximal content of 5% indicated above since the glass transition temperature of the material prepared becomes too low. It is highly recommended for the preparation of the material (lens) of thickness between 1.5 and 20 mm, a polymerization modifier content of about 0.5 wt. %. It has been noted that the colorability and the darkening kinetics of the matrix increase with the amount of polymerization modifier which intervenes. In the same way, when this amount goes up, the mechanical resistance increases and the optical quality improves.

It is obviously appropriate that the polymerization modifier does not destroy the photochromic coloring agent (s) present during the polymerization and/or do not induce a discoloration of the material on its own. The polymerization modifier is advantageously a chain transfer agent. The chain transfer agent can be a non-halogenated chain transfer agent such as a linear alkane thiol or bis-mercapto-ethyl ether. Dodecanethiol may be cited as an example of a linear alkane thiol without being limiting. It is not excluded to use other types of chain transfer agents such as alkane thiols substituted with at least one aryl or alkyl radical or thiophenols. All these compounds are familiar to the person skilled in the art and are commercially available.

The radical polymerization initiator or intervening catalyst (which can be a thermal initiator, a photoinitiator, or a combination of these), must itself be substantially "inert" towards photochromic coloring agent(s) present. The catalyst is generally used at a rate of 0.001 to 1% by weight, preferably from 0.005 to 0.5% by weight, with respect to the weight of the monomers present.

For thermal polymerization, the initiator may preferably be selected from the diazo compounds. These compounds are familiar to the person skilled in the art and arc commercially available. Examples of such diazo compounds are azobisisobutyronitrile (AIBN) and 2,2'-azobis(2-methylbutyronitrile)(AMBN). In the absence of such a catalyst or in the presence of too low an amount of it, it becomes necessary to carry out the copolymerization at a higher temperature and this renders the reaction difficult to control ... In the presence of too great an amount of catalyst, an excess of free radicals may be generated, this excess of free radicals inducing a destruction of the photochromic coloring agent(s) optionally present and an accelerated fatigue of the final material. In this latter hypothesis, the reaction carried out may also accelerate and become difficult to control.

Another way to polymerize the composition is to use UV or visible light. In this process, the pholoinitiator can be selected from molecules known in the field such as described in "Photoinitators for Pigmented Systems" by K. Dictliker / Radiation curing in Polymer Science and Technology: Vol 2; photoinitiating systems - FOUASSIER J.P., RABRCK J.F. Elsevier Applied Science pp 155. Ch3. In this case, as in thermal polymerisation, the photoinitiator must of necessity be substantially "inert" towards photochromic dyes. Examples of useful photoinitiators include benzophenones, thioxanthones, alpha-amino-acetophenone derivatives, acylphosphine oxides, bisacylphosphine oxides and many other such compounds known to those skilled in the art. Specific examples of such compounds include acylphosphates and acyldibenzoxaphine oxide. Acylphosphine oxides can be used either alone or in combination with other classes of photoinitiators such as alpha-hydroxy ketones and benzyldimethyl ketal. One particularly useful example of a photoinitiator is IRGACURE 819 (from CIBA-GEIGY). The two types of polymerization (thermal and photopolymerization) can be used independently or in any combination to obtain the lens.

An original type of (co)polymer matrix has at length been described above within which the photochromic compounds of the invention therefore advantageously intervene, taken alone or advantageously in a mixture. Together with the photochromic compounds,

- at least one other photochromic compound, (*vide supra*),
- and/or, at least one non photochromic coloring agent, this with the aim of adjusting the gray tint in the darkened state,
- and/or, one or more stabilizers, such as an anti-oxidant for example,
- and/or, one or more anti-UV,
- and/or, one or more anti-radicals,
- and/or, one or more deactivators of pholochromic excited states,

may be brought in a manner *per se*.

These additives may notably allow improving the durability of the intervening photochromic compound(s).

The photochromic compounds of another type, non-photochromic coloring agents, stabilizers, are prior art products known to the person skilled in the art.

According to its last object, the present invention relates to finished products or articles (glazings, notably for buildings, locomotives, automobiles; optical devices; ophthalmic or solar articles, notably lenses; decorative articles; solar protection articles; articles useful for storing information ...) which contain an effective quantity of at least one of the photochromic compounds (I) or (II) and advantageously an effective amount of a mixture of the compounds (I) and (II). The articles may notably be constituted, wholly or in part, of a (co)polymer matrix which therefore contains alone or preferably, a mixture of photochromic compounds (I) and (II).

The present invention is illustrated by the following Examples, of synthesis and photochromic validation, of the compounds of the invention.

Examples: 1. Synthesis of compound (I)

Compound (I) (described in the patent application WO-A-98 04937) is obtained by heating a mixture of 1-(p-dimethylaminophenyl)-1-phenyl-2-propyn-1-ol and 5,7-dimethoxy-3-methyl-1-naphthol in refluxing tetrahydrofuran in the presence of p-toluenesulfonic acid; the naphthol having been prepared according to the method described in J. Org. Chem. 1986. vol 51, p271-273 (Sibi *et al*).

2. Synthesis of compound (II)

Compound (II) is synthesized according to the following scheme:

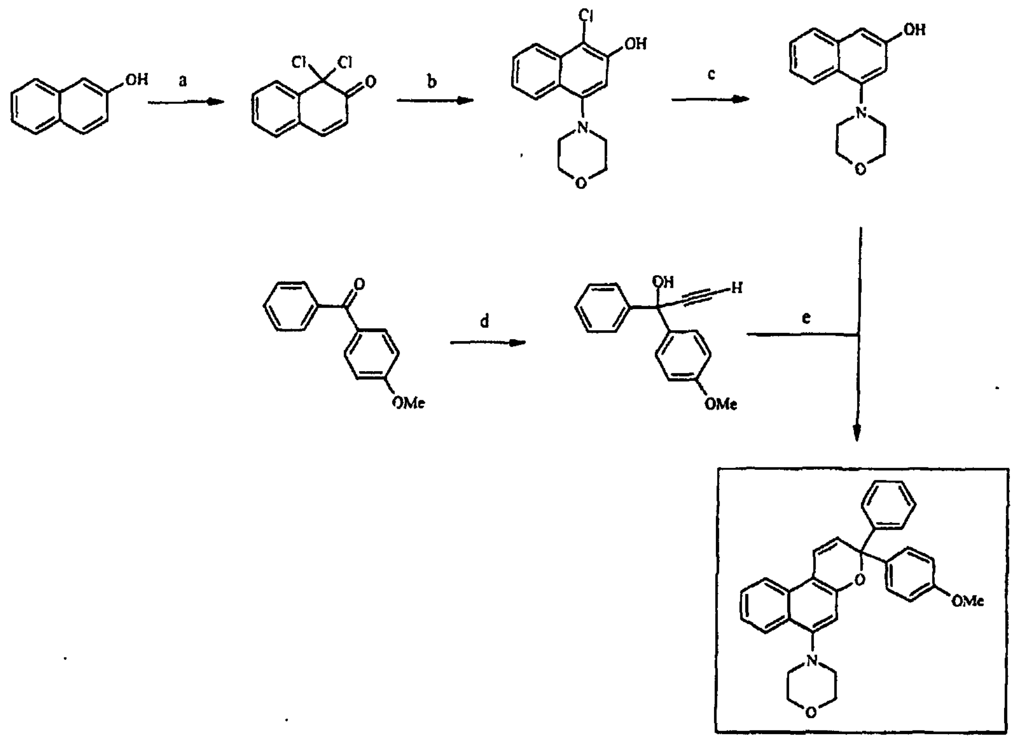


The synthetic route used is essentially that described in the application WO-A- 94 22850.

Step (a) : 101 g of chlorine gas are allowed to react with 100 g of 2-naphthol in 560 ml of acetic acid in the presence of 280 g of sodium acetate according to the experimental protocol described in J. Chem. Soc. 1945, p 280. 144 g of 1,1-dichloronaphtalene-2(1H)-one are obtained.

Step (b) : A mixture constituted of 17.9 g of morpholine and 20.8 g of triethylamine is run into a solution containing 40 g of 1,1-dichloronaphthalene-2(1H)-one in 320 ml of toluene at ambient temperature. After 1.5 hours, the mixture is poured into 200 ml of water. The organic phase is recovered and 300 ml of water followed by a concentrated solution of sodium hydroxide (50 ml) are added. The aqueous phase is recovered and the sodium hydroxide is neutralized by acetic acid which brings about the precipitation of the 1-chloro-4-morpholino-2-naphthol. After filtration, washing with water and drying, 42 g of product are obtained.

Step (c) : in a 0.3 1 steel autoclave, 10.5 g of 1-chloro-4-morpholino-2-naphthol in 150 ml of sodium hydroxide (2N) and 30 ml of ethanol are maintained under a pressure of 3 bar and at 35°C in the presence of 3 g of palladium on charcoal (5%) for 7 hours. The solution is then filtered to remove the catalyst. The aqueous phase is then acidified with acetic acid and then filtered. The precipitate is washed with water and then dried, 7.1 g of 4-morpholino-2-naphthol are thus obtained.

Step (d) : 1-(p-methoxyphenyl)-1-phenyl-2-propyn-1-ol is synthesized from 4-methoxybenzophenone and lithium acetylide (ethylene diamine complex) in DMSO as described in the patent EP-A- 250 193.

Step (e) : 0.91 g of 1-chloro-4-morpholino-2-naphthol are allowed to react with 0.95 g of 1-(p-methoxyphenyl)-1-phenyl-2-propyn-1-ol in 30 ml of THF under reflux for 4 hours in the presence of 0.72 g of p-toluene sulfonic acid. The solution is then neutralized with 30 ml of IN sodium hydroxide and then extracted twice with 30 ml of toluene. The organic phases are evaporated and then the photochrome is separated by chromatography on an alumina column in eluting with a mixture of ethyl acetate/diisopropyl ether (10/90). 250 mg of 3-(p-methoxyphenyl)-3-phenyl-6-morpholino-[3H]-naphtho-[2,1-b]pyran (compound II) are thus obtained. Its structure is confirmed by $^{1}H$ NMR spectroscopy.

3. Synthesis of control compounds (compounds du type (II)):

Compounds C1 to C3 were obtained in a manner analogous to that described above for compound (II).

Structures of compounds (I), (II), C1, C2 and C3:

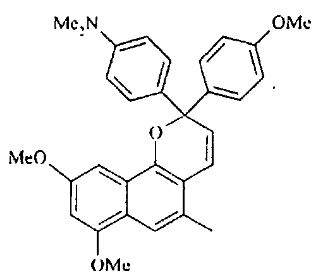

I

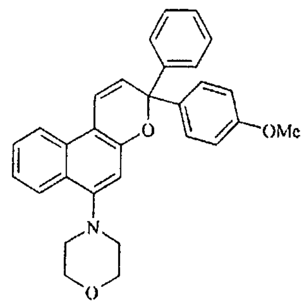

II

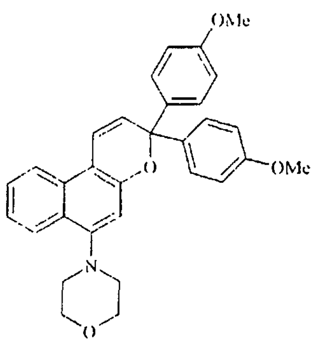

C1

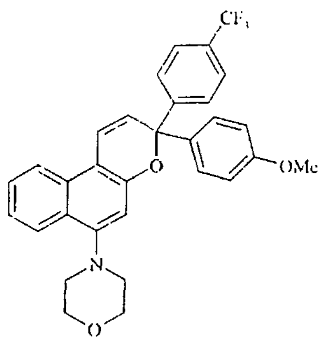

C2

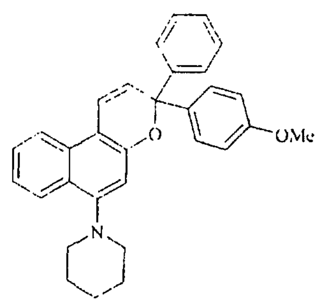

C3

The half-time of fading : $t_{1/2}$, expressed in seconds is specified below for each one of the compounds. This parameter characterizes the kinetics of the return to the initial state (after darkening)(see its exact definition given earlier in the present text with reference to the Table grouping the properties of compound (I)).

| | |
|---|---|
| Compound (I) | 127s |
| Compound (II) | 88s |
| Compound C1 | 52s |
| Compound C2 | 150s |
| Compound C3 | 165s |

The interest of compound (II) over certain of its homologues (C2 and C3) is totally obvious.

4. Preparation of the photochromic matrix :

Step 1 : Synthesis of the alkenic difunctional monomer of formula (B) :

500 g of polyethyleneglycol 600 (Aldrich) are heated at 45 °C in a thermostated reactor under a current of nitrogen. 2.5 g of 4-methoxyphenol (Aldrich) and 3 g of tin dibutyldilaurate (Aldrich) are added into the reactor. 329 g of m-isopropenyl-α-α-dimethylbenzyl isocyanate (m-TMI®) from CYTEC Industries are then added into the reactor at a rate of about 300 g/hour. Once the addition of m-TMI® is complete, the mixture is allowed to stir at 50°C for one

hour. The product obtained is then brought to ambient temperature.

Step 2 : Preparation of the (co)polymer: mixture of (meth)acrylate difunctional monomer of formula (A) and alkenic difunctional monomer of formula (B).

21 g of urethane monomer of the preceding step are mixed with 20.5 g of divinylbenzene (Aldrich), 14 g of benzylmethacrylate (Aldrich) and 44.5 g of tetraethoxy Bisphenol A dimethacrylate (DIACRYL 121 - AKZO).

Step 3 : Preparation of the matrix: addition of photochromic coloring agents (combination of the two coloring agents) and polymerization conditions:

To the mixture of monomers are added the photochromic coloring agents at the rates given in the following Table (the amounts of the coloring agents are expressed in grams per 100 g of mixture of monomers).

| Sample | Compound (I) | Compound (II) | C1 | C2 | C3 |
|---|---|---|---|---|---|
| No 1 | 0.075 | 0.009 | 0 | 0 | 0 |
| No 2 | 0.075 | 0 | 0.015 | 0 | 0 |
| No 3 | 0.075 | 0 | 0 | 0.0075 | 0 |
| No 4 | 0.075 | 0 | 0 | 0 | 0.006 |

0.2 g (by mass) of AMBN 2,2'-azobis(2-methylbutyronitrile) supplied by AKZO (Perkadox®) are dissolved in each composition.

The 2 mm plane samples are then molded between 2 glass plates with a PVC joint. The polymerization is carried out at 60°C for 8 hours and then at 90°C for 2 hours. After processing, the samples are re-baked for 1 hour at 120°C.

5. <u>Results :</u>

The optical transmission between 190 and 900 nm is measured for each sample in the light state (TO) and then in the dark state (TD15) after 15 minutes exposure to UV-visible under a filtered Xenon source (distribution near to AM2 Moon). From the transmission spectra, the chromatic co-ordinates X, Y, Z and L, a, b were calculated according to the ASTM E308-90 method. The yellow index in the light state was calculated from these co-ordinates according to the ASTM D1925-70 method. The photochromic properties of the samples are given in the following Tables.

| Sample | *T0 | **TD15 (25 °C) | **TD15 (40 °C) |
|---|---|---|---|
| No 1 | 82.6 % | 10.7 % | 34.4 % |
| No 2 | 84.4 % | 13.8 % | 37.7 % |
| No 3 | 82.8 % | 10.8 % | 33.4 % |
| No 4 | 83.1 % | 11.4 % | 32.9 % |

*T0 = transmission measured at 560 nm in the light state

**TD15 = transmission measured at 560 nm after 15 minutes' exposure.

| Sample | Yellow index | a d15 25 °C | b d15 25 °C | a d15 40 °C | b d15 40 °C | Δa $a_{25}$-$a_{40}$ | Δb $b_{25}$-$b_{40}$ |
|---|---|---|---|---|---|---|---|
| No 1 | 9.8 | 0.2 | -6.8 | -0.6 | -0.8 | 0.8 | - 6.0 |
| No 2 | 12.7 | 4.0 | - 2.8 | 2.0 | 1.4 | 2.0 | -4.2 |
| No 3 | 9.9 | 0.6 | -10.3 | -0.7 | -3.6 | 1.3 | -6.7 |
| No 4 | 10.6 | 4.0 | -9.6 | -0.8 | -2.1 | 1.8 | -7.5 |

a d15 and b d15 designate the chromatic co-ordinates in the darkened state after 15 minutes' exposure. Δa and Δb represent the variations in color between 25 and 40°C.

It is demonstrated by these measurements that Sample No. 2 has a yellow index which is too high and a colorability (TD15) at 40°C which is much lower than the other examples. The colors in the darkened state of this example are not constant: a purple tendency at 25°C and brown at 40°C.

In the darkened state, Samples No. 3 and 4 are gray with a purple hint at 25°C and gray with a hint of blue at 40°C. Sample No. 1 with a lower yellow index is gray with a hint of blue at 25°C and neutral gray at 40°C.

In summary, Sample No. 1 is that which had the best color consistency between 25 and 40°C (the deviations Δa and Δb are lower for this sample) with a high colorability at 40°C and a relatively low yellow index.

## Claims

1. A photochromic composition incorporating

   - \+ 2-(p-dimethylaminophenyl)-2-(p-methoxyphenyl)-5-methyl-7,9-dimethoxy-[2H]-naphtho[1,2-b]pyran (compound (I)),
     and
   - \+ 3-(p-methoxyphenyl)-3-phenyl-6-morpholino-3H-naphtho-[2,1-b]pyran (compound (II)).

2. The photochromic composition according to claim 1, **characterized in that**, within it, the 2-(p-dimethylaminophenyl)-2-(p-methoxyphenyl)-5-methyl-7,9-dimethoxy-[2H]-naphtho[1,2-b]pyran (compound I)/ 3-(p-methoxyphenyl)-3-phenyl-6-morpholino-3H-naphtho-[2,1-b]pyran (compound II) weight ratio is between 7 and 10, advantageously between 8 and 9.

3. Joint use of 2-(p-dimethylaminophenyl)-2-(p-methoxyphenyl)-5-methyl-7,9-dimethoxy-[2H]-naphtho[1,2-b]pyran (compound I) and 3-(p-methoxyphenyl)-3-phenyl-6-morpholino-3H-naphtho-[2,1-b]pyran (compound II) as photochromic agents ; the two photochromic agents being advantageously used in a compound (I) / compound (II) weight ratio between 7 and 10.

4. 3-(p-methoxyphenyl)-3-phenyl-6-morpholino-3H-naphtho-[2,1-b]pyran.

5. A (co)polymer matrix **characterized in that** it comprises the compound according to claim 4 and advantageously a mixture of the compounds according to one of claims 1 or 2.

6. The (co)polymer matrix according to claim 5, **characterized in that** the (co)polymer is selected from the following list:

   a) optionally halogenated alkyl, cycloalkyl, aryl or aralkyl mono-, di-, tri- or tetraacrylate or mono-, di-, tri- or tetramethacrylate or having at least one ether and/or ester and/or carbonate and/or carbamate and/or thiocarbamate and/or urea and/or amide group,
   b) polystyrene, polyether, polyester, polycarbonate, polycarbamate, polyepoxy, polyurea, polyurethane, polythiourethane, polysiloxane, polyacrylonitrile, polyamide, aliphatic or aromatic polyester, vinylic polymer, cellulose acetate, cellulose triacetate , cellulose acetate-propionate or polyvinylbutyral,
   c) copolymers of two or more types of monomer or mixtures of polymers mentioned above,
   d) copolymers obtained by radical polymerization of a composition comprising a mixture of at least one or more difunctional monomers of type (a) and one or more difunctional monomers of type (b):

   * the difunctional monomer(s) of type (a) having one or the other of the formulae (A) and (A') below:

\+ formula (A) :

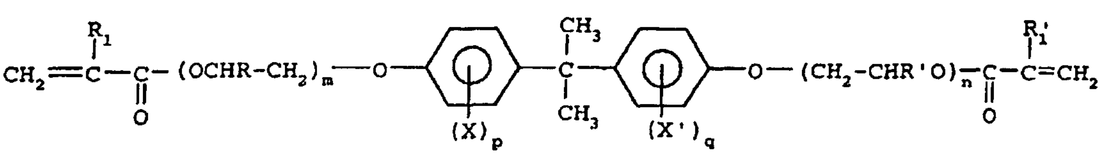

in which:

- $R_1$, $R'_1$, R and R', identical or different, independently are a hydrogen or a methyl group;
- m and n are, independently, integers between 0 and 4 inclusive ; and are advantageously independently equal to 1 or 2 ;
- X and X', identical or different, are a halogen and preferably represent chlorine and/or bromine;
- p and q are, independently, integers between 0 and 4 inclusive ;

+ formula (A') :

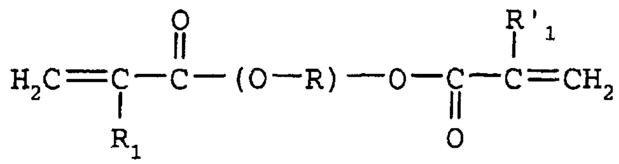

in which :
- $R_1$ and $R'_1$, identical or different, independently are a hydrogen or a methyl group;
- R is a linear or branched alkyl radical having from 2 to 8 carbon atoms, a cycloalkyl radical having from 3 to 6 carbon atoms, an ether radical of formula (R'-O-R") in which R' and R", identical or different, independently are a linear or branched alkyl radical having from 2 to 4 carbon atoms;

* the difunctional monomer(s) of type (b) - long chain alkenic difunctional oligomer - being of one or the other of formulae (B), (B') and (B") below:

+ formula (B) :

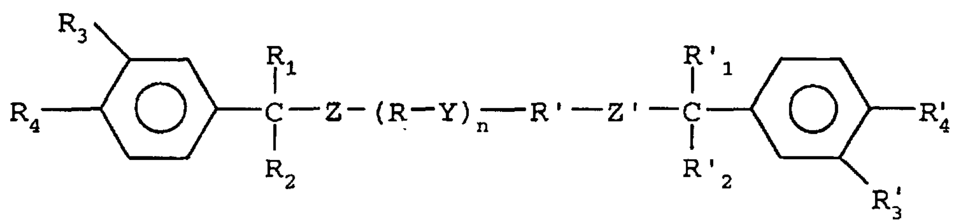

in which:

- $R_1$, $R'_1$, $R_2$ and $R'_2$, identical or different, independently are hydrogen or a linear or branched alkyl radical, advantageously linear, having from 1 to 4 carbon atoms ; and correspond particularly advantageously to a methyl group;
- $R_3$ and $R_4$, different, are independently one hydrogen and the other an alkenyl radical having from 2 to 6 carbon atoms, advantageously from 2 to 4 carbon atoms and particularly advantageously an isopropenyl radical;
- $R'_3$ and $R'_4$, different, are independently one hydrogen and the other an alkenyl radical having from 2 to 6 carbon atoms, advantageously from 2 to 4 carbon atoms and particularly advantageously an isopropenyl radical;
- Z represents a carbamate function (-NH-CO-O-), a thiocarbamate function (-NH-CO-S-) or a urea function (-NH-CO-NH-) ;
- Z', independent from Z and advantageously respectively with respect to Z, represents a carbamate function (-O-CO-NH-), a thiocarbamate function (-S-CO-NH-) or a urea function (-NH-CO-NH-);
- R' represents a linear or branched alkyl radical having from 2 to 4 carbon atoms ;
- R, identical or different when $n \geq 2$, is a linear or branched alkyl radical having from 2 to 4 carbon atoms ;
- Y, identical or different when $n \geq 2$, is oxygen or sulfur ;
- n is an integer defined in such a way that the total number of carbon atoms contained in the long chain situated between the two motifs Z and Z' is at least equal to 18 and is advantageously between

18 and 112 inclusive ;

+ formula (B') :

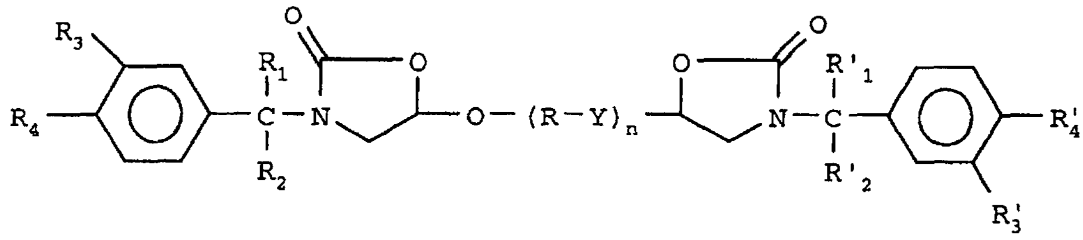

in which:

- $R_1$, $R_2$, $R_3$, $R_4$, $R'_1$, $R'_2$, $R'_3$, $R'_4$, R and Y are such as defined above with reference to formula (B);
- n is an integer defined in such a way that the total number of carbon atoms contained in the long chain of the motif $(R-Y)_n$ is at least equal to 22 and is advantageously between 22 and 104 inclusive;

+ formula (B"):

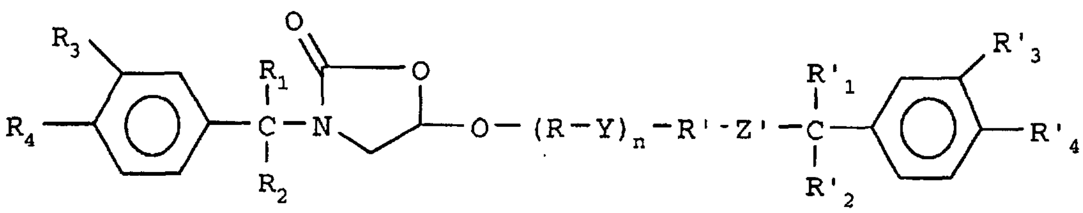

in which:

- $R_1$, $R_2$, $R_3$, $R_4$, $R'_1$, $R'_2$ $R'_3$, $R'_4$, R, R' and Y are such as defined above with reference to formula (B);
- Z' is a carbamate function (-O-CO-NH-) or Z' is a thiocarbamate function (-S-CO-NH-); advantageously Z' is a carbamate function;
- n is an integer defined in such a way that the total number of carbon atoms contained in the long chain of the motif $(R-Y)_n$-R' is at least equal to 22 and is advantageously between 22 and 104 inclusive.

**7.** The (co)polymer matrix according to claim 6, **characterized in that** the copolymer is obtained by radical polymerization of a composition such as defined in point d); the difunctional monomer(s) of type (a) having formula (A) in which:

- $R_1$ and $R'_1$, identical, are a methyl group ;
- R and R', identical, are hydrogen or a methyl group ;
- m and n independently are 1 or 2 ;
- p and q are identical and equal to 0 ;

and advantageously R and R' represent hydrogen and m = n = 2;
and/or the symmetrical long-chain alkenic difunctional oligomer(s) of formula (B) and/or of formula (B') and/or of formula (B"), in which $R_1$, $R'_1$, $R_2$, $R'_2$ are identical and represent a methyl group, $R_3$ and $R'_3$, identical, represent an isopropenyl group and $R_4$ and $R'_4$, identical, are hydrogen.

**8.** The (co)polymer matrix according to one of claims 6 or 7, **characterized in that** the copolymer is obtained by radical polymerization of a composition such as defined in point d) of claim 6; the alkenic difunctional oligomer(s) of formula (B) and/or of formula (B') and/or of formula (B"), formulae in which the long polyoxyalkylene and/or polymercaptoalkylene chain, represented by the motif $(R-Y)_n$-R' in the case of oligomer(s) of formula (B) or of formula (B"), or by the motif $(R-Y)_n$ in the case of oligomer(s) of formula (B'), having a molecular mass between 500 $g.mol^{-1}$ and 2,000 $g.mol^{-1}$; and advantageously the molecular mass of said long chain being at least 600 g.

mol$^{-1}$ and lower than 900 g.mol$^{-1}$.

**9.** The (co)polymer matrix according to any one of claims 6 to 8, **characterized in that** the copolymer is obtained by radical polymerization of a composition such as defined in point d) of claim 6; the difunctional oligomer(s) of type b) having formula (B):
in which:

- Z and Z' are urea functions (-NH-CO-NH-);
- $R_1$, $R_2$, $R_3$, $R_4$, $R'_1$, $R'_2$, $R'_3$ and $R'_4$ are such as defined in claim 7 ;
- R' represents an ethylene or propylene group;
- n is an integer equal to 13 or 19 which defines a total number of carbon atoms, between Z and Z', equal to 28 or 40 when $(R\text{-}Y)_n$ is a polyoxyethylene chain; or n is an integer equal to 10 or 14 which defines a total number of carbon atoms, between Z and Z', equal to 33 or 45 when $(R\text{-}Y)_n$ is a polyoxypropylene chain; or n is an integer between the lower limits (n is between 10 and 13 inclusive) and higher limit values (n is between 14 and 19 inclusive), when $(R\text{-}Y)_n$ is a mixed polyoxyethylene / polyoxypropylene chain,

or in which:

- Z and Z' are carbamate functions of formula (-NH-CO-O-) and (-O-CO-NH-) respectively.
- $R_1$, $R_2$, $R_3$, $R_4$, $R'_1$, $R'_2$, $R'_3$ and $R'_{4°}$ are such as defined in claim 7 ;
- R' represents an ethylene group;
- $(R\text{-}Y)_n$ represents a long chain polyoxyethylene ;
- n is an integer equal to 13 or 19 which defines a total number of carbon atoms contained in the long chain situated between the two motifs Z and Z', equal to 28 or 40.

**10.** The (co)polymer matrix according to any one of claims 6 to 9, **characterized in that** the copolymer is obtained by radical polymerization of a composition such as defined in point d) of claim 6; the amount of monomer(s) of type (a) being between 40 and 99 parts by weight, for 100 parts by weight of the mixture of monomers of type (a) and (b).

**11.** The (co)polymer matrix according to claim 10, further **characterized in that** it contains an effective amount of a radical polymerization initiator.

**12.** The (co)polymer matrix according to claim 11, wherein the radical polymerization initiator is selected from the group consisting of a thermal initiator, a photoinitiator, and a mixture of these.

**13.** A finished product of the glazing, optical device, ophthalmic or solar article type, consisting advantageously of a lens constituted wholly or in part of a matrix according to any one of claims 5 to 12 and/or containing the compound according to claim 4, and advantageously a mixture of the compounds according to one of claims 1 or 2.

## Patentansprüche

**1.** Photochrome Zusammensetzung, die umfasst

+ 2-(p-Dimethylaminophenyl)-2-(p-methoxyphenyl)-5-methyl-7,9-dimethoxy-[2H]-naphtho[1,2-b]pyrari (Verbindung(I)),
  und
+ 3-(p-Methoxyphenyl)-3-phenyl-6-morpholino-3H-naphtho-[2,1-b]pyran (Verbindung (II)).

**2.** Photochrome Zusammensetzung nach Anspruch 1, **dadurch gekennzeichnet, dass** in ihr das Gewichtsverhältnis zwischen 2-(p-Dimethylaminophenyl)-2-(p-methoxyphenyl)-5-methyl-7,9-dimethoxy-[2H]-naphtho[1,2-b]pyran (Verbindung I) und 3-(p-Methoxyphenyl)-3-phenyl-6-morpholino-3H-naphtho-[2,1-b]pyran (Verbindung II) zwischen 7 und 10, zweckmäßig zwischen 8 und 9, liegt.

**3.** Gemeinsame Verwendung von 2-(p-Dimethylaminophenyl)-2-(p-methoxyphenyl)-5-methyl-7,9-dimethoxy-[2H]-naphtho[1,2-b]pyran (Verbindung (I)) und 3-(p-Methoxyphenyl)-3-phenyl-6-morpholino-3H-naphtho-[2,1-b]pyran (Verbindung (II)) als photochrome Agentien, die zweckmäßig in einem Gewichtsverhältnis zwischen der Verbindung (I) und der Verbindung (II) zwischen 7 und 10 verwendet werden.

**4.** 3-(p-Methoxyphenyl)-3-phenyl-6-morpholino-3H-naphtho-[2,1.b]pyran.

**5.** (Co)Polymer-Matrix, **dadurch gekennzeichnet, dass** sie die Verbindung nach Anspruch 4 und zweckmäßig eine Mischung der Verbindungen nach einem der Ansprüche 1 oder 2 umfasst.

**6.** (Co)Polymer-Matrix nach Anspruch 5, **dadurch gekennzeichnet, dass** das (Co)Polymer ausgewählt ist aus der folgenden Liste:

a) gegebenenfalls halogeniertem Alkyl-, Cycloalkyl-, Aryl- oder Aralkyl-mono-, -di-, -tri- oder -tetraacrylat oder -mono-, -di-, -tri- oder -tetramethacrylat oder einem solchen, das mindestens eine Ether- und/oder Esterund/oder Carbonat- und/oder Carbamat- und/oder Thiocarbamat- und/oder Harnstoff- und/oder Amidgruppe aufweist,
b) Polystyrol, Polyether, Polyester, Polycarbonat, Polycarbamat, Polyepoxy, Polyharnstoff, Polyurethan, Polythiourethan, Polysiloxan, Polyacrylnitril, Polyamid, aliphatischem oder aromatischem Polyester, Vinyl-Polymer, Celluloseacetat, Cellulosetriacetat, Celluloseacetatpropionat oder Polyvinylbutyral,
c) Copolymeren von zwei oder mehr Monomer-Typen oder Mischungen der oben genannten Polymeren,
d) Copolymeren, die hergestellt worden sind durch radikalische Polymerisation einer Zusammensetzung, die eine Mischung aus mindestens einem oder mehr difunktionellen Monomeren des Typs (a) und einem oder mehr difunktionellen Monomeren des Typs (b) umfasst:

* wobei das (die) difunktionelle(n) Monomer(en) vom Typ (a) eine der nachstehend angegebenen Formeln (A) und (A') hat (haben):

+ Formel (A):

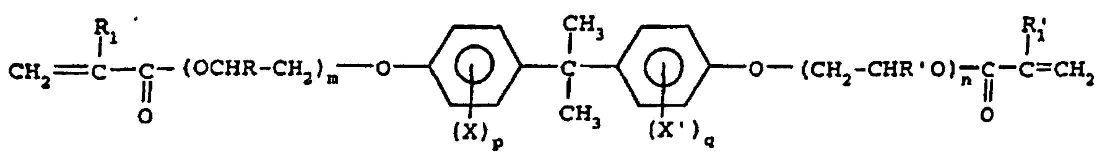

worin bedeuten:

- $R_1$, $R'_1$, R und R', die gleich oder verschieden sind, unabhängig voneinander ein Wasserstoffatom oder eine Methylgruppe;
- m und n unabhängig voneinander ganze Zahlen zwischen 0 und einschließlich 4 und zweckmäßig unabhängig voneinander die Zahl 1 oder 2;
- X und X', die gleich oder verschieden sind, Halogen, vorzugsweise Chlor und/oder Brom; und
- p und q unabhängig voneinander ganze Zahlen zwischen 0 und einschließlich 4;

+ Formel (A')

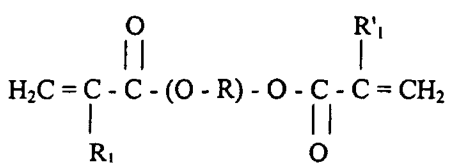

worin bedeuten:

- $R_1$ und $R'_1$, die gleich oder verschieden sind, unabhängig voneinander ein Wasserstoffatom oder eine Methylgruppe;
- R einen linearen oder verzweigten Alkylrest mit 2 bis 8 Kohlenstoffatomen, einen Cycloalkylrest mit 3 bis 6 Kohlenstoffatomen, einen Etherrest der Formel (R'-O-R"), worin R' und R", die gleich oder verschieden sind, unabhängig voneinander für einen linearen oder verzweigten Alkylrest mit 2 bis 4 Kohlenstoffatomen stehen; und

* das (die) difunktionelle(n) Monomer(en) des Typs (b) - ein langkettiges Alken-difunktionelles Oligomer - eine der nachstehenden Formeln (B), (B') und (B") hat (haben): + Formel (B):

+ Formel (B):

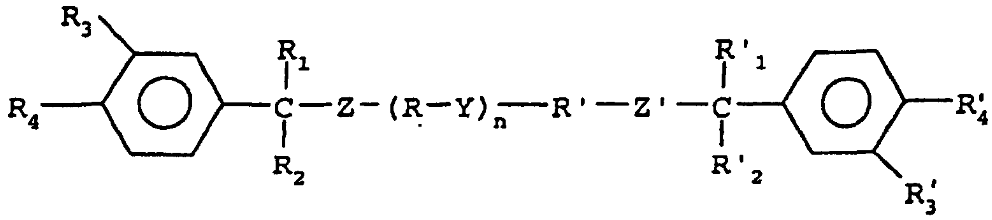

worin bedeuten:

- $R_1$, $R'_1$, $R_2$ und $R'_2$, die gleich oder verschieden sind, unabhängig voneinander ein Wasserstoffatom oder einen linearen oder verzweigten Alkylrest, zweckmäßig einen linearen Alkylrest mit 1 bis 4 Kohlenstoffatomen und besonders zweckmäßig eine Methylgruppe;
- $R_3$ und $R_4$, die voneinander verschieden sind, jeweils unabhängig voneinander, der eine ein Wasserstoffatom und der andere einen Alkenylrest mit 2 bis 6 Kohlenstoffatomen, zweckmäßig mit 2 bis 4 Kohlenstoffatomen und besonders vorteilhaft einen Isopropenylrest;
- $R'_3$ und $R'_4$, die verschieden sind, jeweils unabhängig voneinander, der eine ein Wasserstoffatom und der andere einen Alkenylrest mit 2 bis 6 Kohlenstoffatomen, zweckmäßig mit 2 bis 4 Kohlenstoffatomen und besonders zweckmäßig einen Isopropenylrest;
- Z eine Carbamat-Funktion (-NH-CO-O-), eine Thiocarbamat-Funktion (-NH-CO-S-) oder eine Harnstofffunktion (-NH-CO-NH-);
- Z' unabhängig von Z und zweckmäßig jeweils in bezug auf Z, eine Carbamat-Funktion (-O-CO-NH-), eine Thiocarbamat-Funktion (-S-CO-NH-) oder eine Harnstoff-Funktion (-NH-CO-NH-);
- R' einen linearen oder verzweigten Alkylrest mit 2 bis 4 Kohlenstoffatomen;
- R, identisch oder verschieden, wenn $n \geq 2$, einen linearen oder verzweigten Alkylrest mit 2 bis 4 Kohlenstoffatomen;
- Y identisch oder verschieden, wenn $n \geq 2$, Sauerstoff oder Schwefel;
- n eine ganze Zahl, die so definiert ist, dass die Gesamtanzahl der Kohlenstoffatome, die in der zwischen den beiden Motiven Z und Z' angeordneten langen Kette enthalten sind, mindestens 18 beträgt und zweckmäßig zwischen 18 und einschließlich 112 liegt;

+ Formel (B'):

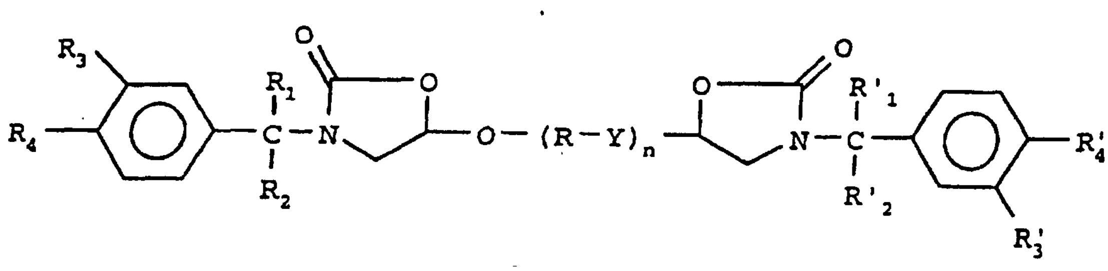

worin:

- $R_1$, $R_2$, $R_3$, $R_4$, $R'_1$, $R'_2$, $R'_3$, $R'_4$, R und Y die gleichen Bedeutungen haben wie oben in bezug auf die Formel (B) angegeben;
- n eine ganze Zahl darstellt, die so definiert ist, dass die Gesamtanzahl der Kohlenstoffatome, die in der langen Kette des Motivs $(R\text{-}Y)_n$ enthalten sind, mindestens 22 beträgt und zweckmäßig zwischen 22 und einschließlich 104 liegt;

+ Formel (B''):

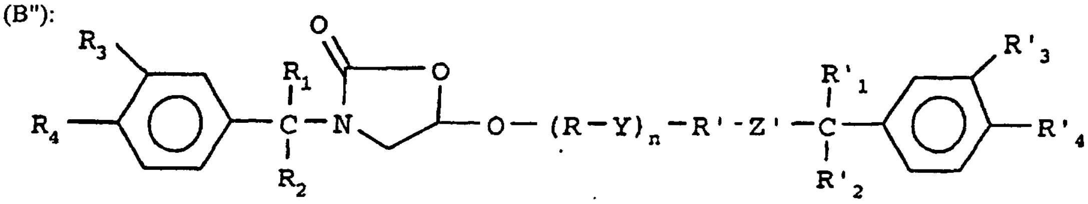

worin:

- $R_1$, $R_2$, $R_3$, $R_4$, R'1, $R'_2$, $R'_3$, $R'_4$, R, R' und Y so definiert sind wie oben in bezug auf die Formel (B) angegeben;
- Z' eine Carbamat-Funktion (-O-CO-NH-) oder eine Thiocarbamat-Funktion (-S-CO-NH-), zweckmäßig eine Carbamat-Funktion, darstellt;
- n für eine ganze Zahl steht, die so definiert ist, dass die Gesamtanzahl der Kohlenstoffatome, die in der langen Kette des Motivs $(R\text{-}Y)_n\text{-}R'$ enthalten sind, mindestens 22 beträgt und zweckmäßig zwischen 22 und einschließlich 104 liegt.

**7.** (Co)Polymer-Matrix nach Anspruch 6, **dadurch gekennzeichnet, dass** das Copolymer hergestellt worden ist durch radikalische Polymerisation einer Zusammensetzung, wie sie unter Punkt (d) definiert ist, des (der) difunktionellen Monomers (Monomeren) des Typs (a), das (die) die Formel (A) hat (haben), worin bedeuten:

- $R_1$ und $R'_1$, die gleich sind, eine Methylgruppe;
- R und R', die gleich sind, ein Wasserstoffatom oder eine Methylgruppe;
- m und n unabhängig voneinander die Zahl 1 oder 2;
- p und q, die gleich sind, die Zahl 0; und

worin zweckmäßig R und R' für Wasserstoff stehen und m = n = 2; und/oder des (der) symmetrischen langkettigen Alken-difunktionelle(n) Oligomers (Oligomeren) der Formel (B) und/oder der Formel (B') und/oder der Formel (B''), worin $R_1$, $R'_1$, $R_2$, $R'_2$ gleich sind und für eine Methylgruppe stehen, $R_3$ und $R'_3$ gleich sind und für eine Isopropenylgruppe stehen,und $R_4$ und $R'_4$ gleich sind und für Wasserstoff stehen.

**8.** (Co)Polymer-Matrix nach einem der Ansprüche 6 oder 7, **dadurch gekennzeichnet, dass** das Copolymer hergestellt worden ist durch radikalische Polymerisation einer Zusammensetzung, wie sie unter Punkt (d) des Anspruchs 6 definiert ist, des (der) Alken-difunktionellen Oligomers (Oligomeren) der Formel (B) und/oder der Formel (B') und/oder der Formel (B''), in denen die lange Polyoxyalkylenkette und/oder die lange Polymercaptoalkylenkette, dargestellt durch das Motiv $(R\text{-}Y)_n\text{-}R'$ im Falle des (der) Oligomer(en) der Formel (B) oder der Formel (B''), oder dargestellt durch das Motiv $(R\text{-}Y)_n$ im Falle des (der) Oligomer(en) der Formel (B'), eine Molekularmasse zwischen 500 und 2000 $g.mol^{-1}$ aufweist, wobei die Molekularmasse der genannten langen Kette zweckmäßig mindestens 600 $g.mol^{-1}$ und weniger als 900 $g.mol^{-1}$ beträgt.

**9.** (Co)Polymer-Matrix nach einem der Ansprüche 6 bis 8, **dadurch gekennzeichnet, dass** das Copolymer hergestellt worden ist durch radikalische Polymerisation einer Zusammensetzung, wie sie unter Punkt (d) des Anspruchs 6 definiert ist, und des (der) difunktionellen Oligomers (Oligomeren) vom Typ (b) der Formel (B), worin:

- Z und Z' Harnstoff-Funktionen (-NH-CO-NH-) darstellen;
- $R_1$, $R_2$, $R_3$, $R_4$, $R'_1$, $R'_2$, $R'_3$ und $R'_4$ so definiert sind wie in Anspruch 7;
- R' eine Ethylen- oder Propylengruppe darstellt;
- n die ganze Zahl 13 oder 19, welche die Gesamtanzahl der Kohlenstoffatome zwischen Z und Z' definiert, die ganze Zahl 28 oder 40, wenn $(R\text{-}Y)_n$ eine Polyoxyethylenkette ist; oder die ganze Zahl 10 oder 14, welche die Gesamtanzahl der Kohlenstoffatome zwischen Z und Z' definiert, die ganze Zahl 33 oder 45, wenn $(R\text{-}Y)_n$ eine Polyoxypropylenkette ist, oder eine ganze Zahl zwischen den unteren Grenzwerten (n liegt zwischen 10 und einschließlich 13) und den oberen Grenzwerten (n liegt zwischen 14 und einschließlich 19), wenn $(R\text{-}Y)_n$ eine gemischte Polyoxyethylen/Polyoxypropylenkette ist, darstellt oder

worin:

- Z und Z' Carbamat-Funktionen der jeweiligen Formel (-NH-CO-O-) und (-O-CO-NH-) darstellen,

- $R_1$, $R_2$, $R_3$, $R_4$, $R'_1$, $R'_2$, $R'_3$ und $R'_4$ so definiert sind wie in Anspruch 7;
- R' eine Ethylengruppe darstellt;
- $(R-Y)_n$ ein langkettiges Polyoxyethylen darstellt;
- n die ganze Zahl 13 oder 19, welche die Gesamtanzahl der Kohlenstoffatome definiert, die in der langen Kette enthalten sind, die zwischen den beiden Motiven Z und Z' angeordnet ist, oder die ganze Zahl 28 oder 40 darstellt.

**10.** (Co)Polymer-Matrix nach einem der Ansprüche 6 bis 9, **dadurch gekennzeichnet, dass** das Copolymer hergestellt worden ist durch radikalische Polymerisation einer Zusammensetzung, wie sie unter Punkt (d) des Anspruchs 6 definiert ist, wobei die Menge des (der) Monomer (Monomeren) vom Typ (a) zwischen 40 und 99 Gew.-Teilen auf 100 Gew.-Teile der Mischung von Monomeren des Typs (a) und (b) liegt.

**11.** (Co)Polymer-Matrix nach Anspruch 10, **dadurch gekennzeichnet, dass** sie außerdem eine wirksame Menge eines radikalischen Polymerisationsinitiators enthält.

**12.** (Co)Polymer-Matrix nach Anspruch 11, in der der radikalische Polymerisationsinitiator ausgewählt ist aus der Gruppe, die besteht aus einem thermischen Initiator, einem Photoinitiator und einer Mischung davon.

**13.** Fertiges Produkt vom Typ eines Verglasungs-, optischen, ophthalmischen oder Solar-Gegenstandes, der zweckmäßig besteht aus einer Linse, die vollständig oder teilweise aus einer Matrix nach einem der Ansprüche 5 bis 12 aufgebaut ist und/oder die Verbindung nach Anspruch 4 und zweckmäßig eine Mischung der Verbindungen nach einem der Ansprüche 1 oder 2 enthält.

## Revendications

**1.** Composition photochromique incorporant du

+ 2-(p-diméthylaminophényl)-2-(p-méthoxyphényl)-5-méthyl-7,9-diméthoxy -[2H]-naphto[1,2-b]pyrane (composé (I)),
  et du
+ 3-(p-méthoxyphényl)-3-phényl-6-morpholino-3H-naphto-[2,1-b]pyrane (composé (II)).

**2.** Composition photochromique selon la revendication 1, **caractérisée en ce que**, en son sein, le rapport pondéral 2-(p-diméthylaminophényl)-2-(p-méthoxyphényl)-5-méthyl-7,9-diméthoxy-[2H]-naphto[1,2-b]pyrane (composé I)/ 3-(p-méthoxyphényl)-3-phényl-6-morpholino-3H-naphto-[2,1-b]pyrane (composé II) est compris entre 7 et 10, avantageusement entre 8 et 9.

**3.** Utilisation conjointe, à titre d'agents photochromes, du 2-(p-diméthylarninophényl)-2-(p-méthoxyphényl)-5-méthyl-7,9-diméthoxy-[2H]-naphto[1,2-b]pyrane (composé I) et du 3-(p-méthoxyphényl)-3-phényl-6-morpholino-3H-naphto-[2,1-b]pyrane (composé II); lesdits deux agents photochromes étant avantageusement utilisés dans un rapport pondéral composé (I) / composé (II) compris entre 7 et 10.

**4.** Le 3-(p-méthoxyphényl)-3-phényl-6-morpholino-3H-naphto-[2,1-b]pyrane.

**5.** Matrice (co)polymère **caractérisée en ce qu'**elle comprend le composé selon la revendication 4 et avantageusement un mélange des composés selon l'une des revendications 1 ou 2.

**6.** Matrice (co)polymère selon la revendication 5, **caractérisée en ce que** ledit (co)polymère est choisi dans la liste suivante :

a) mono-, di-, tri- ou tétraacrylate ou mono-, di-, tri- ou tétraméthacrylate d'alkyle, de cycloalkyle, d'aryle ou d'aralkyle éventuellement halogéné ou comportant au moins un groupement éther et/ou ester et/ou carbonate et/ou carbamate et/ou thiocarbamate et/ou urée et/ou amide,
b) polystyrène, polyéther, polyester, polycarbonate, polycarbamate, polyépoxy, polyurée, polyuréthane, polythiouréthane, polysiloxane, polyacrylonitrile, polyamide, polyester aliphatique ou aromatique, polymère vinylique, acétate de cellulose, triacétate de cellulose,' acétate-propionate de cellulose ou polyvinylbutyral,
c) copolymères de deux ou plusieurs types de monomères ou mélanges de polymères visés supra,

d) copolymères obtenus par polymérisation, par voie radicalaire, d'une composition comprenant un mélange d'au moins un ou plusieurs monomères difonctionnels de type (a) et un ou plusieurs monomères difonctionnels de type (b):

* le(s) monomère(s) difonctionnel(s) de type (a) répondant à l'une ou l'autre des formules (A) et (A') ci-après :

+ formule (A) :

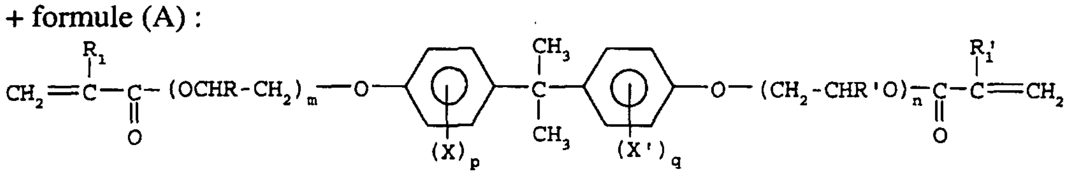

dans laquelle :

- $R_1$, $R'_1$, R et R', identiques ou différents, sont indépendamment un hydrogène ou un groupe méthyle ;
- m et n sont, indépendamment, des entiers compris entre 0 et 4 inclus ; et sont avantageusement indépendamment égaux à 1 ou 2 ;
- X et X', identiques ou différents, sont un halogène et représentent de préférence un chlore et/ou un brome ;
- p et q sont, indépendamment, des entiers compris entre 0 et 4 inclus ;

+ formule (A') :

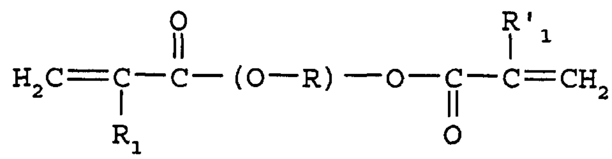

dans laquelle :
- $R_1$ et $R'_1$, identiques ou différents, sont indépendamment un hydrogène ou un groupe méthyle ;
- R est un radical alkyle linéaire ou ramifié comportant de 2 à 8 atomes de carbone, un radical cycloalkyle comportant de 3 à 6 atomes de carbone, un radical éther de formule (R'-O-R") dans laquelle R' et R", identiques ou différents, sont indépendamment un radical alkyle linéaire ou ramifié comportant de 2 à 4 atomes de carbone;

* le(s) monomère(s) difonctionnel(s) de type (b) - oligomère difonctionnel alcénique à chaîne longue - répondant à l'une ou l'autre des formules (B), (B') et (B") ci-après:

+ formule (B) :

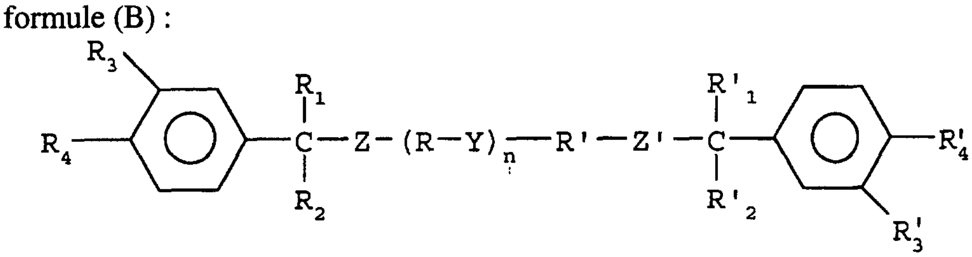

dans laquelle :

- $R_1$, $R'_1$, $R_2$ et $R'_2$, identiques ou différents, sont indépendamment l'hydrogène ou un radical alkyle linéaire ou ramifié, avantageusement linéaire, comportant de 1 à 4 atomes de carbone ; et correspondent de façon particulièrement avantageuse à un groupe méthyle ;
- $R_3$ et $R_4$, différents, sont indépendamment l'un l'hydrogène et l'autre un radical alcényle comportant de 2 à 6 atomes de carbone, avantageusement de 2 à 4 atomes de carbone et d'une façon particulièrement avantageuse un radical isopropényle ;

- $R'_3$ et $R'_4$, différents, sont indépendamment l'un l'hydrogène et l'autre un radical alcényle comportant de 2 à 6 atomes de carbone, avantageusement de 2 à 4 atomes de carbone et d'une façon particulièrement avantageuse un radical isopropényle ;
- Z représente une fonction carbamate (-NH-CO-O-), une fonction thiocarbamate (-NH-CO-S-) ou une fonction urée (-NH-CO-NH-) ;
- Z', indépendamment de Z et de façon avantageuse respectivement par rapport à Z, représente une fonction carbamate (-O-CO-NH-), une fonction thiocarbamate (-S-CO-NH-) ou une fonction urée (-NH-CO-NH-) ;
- R' représente un radical alkyle linéaire ou ramifié comportant de 2 à 4 atomes de carbone ;
- R, identique ou différent quand $n \geq 2$, est un radical alkyle linéaire ou ramifié comportant de 2 à 4 atomes de carbone ;
- Y, identique ou différent quand $n \geq 2$, est l'oxygène ou le soufre ;
- n est un entier défini de tel sorte que le nombre total d'atomes de carbone, contenus dans la chaîne longue située entre les deux motifs Z et Z', soit au moins égal à 18 et d'une façon avantageuse soit compris entre 18 et 112 inclus ;

+ formule (B') :

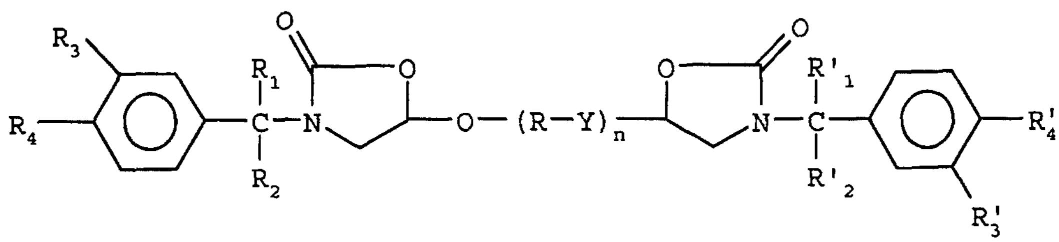

dans laquelle :

- $R_1$, $R_2$, $R_3$, $R_4$, $R'_1$, $R'_2$, $R'_3$, $R'_4$, R; et Y sont tels que définis ci-dessus en référence à la formule (B);
- n est un entier défini de tel sorte que le nombre total d'atomes de carbone, contenus dans la chaîne longue de motif $(R\text{-}Y)_n$, soit au moins égal à 22 et d'une façon avantageuse soit compris entre 22 et 104 inclus;

+ formule (B") :

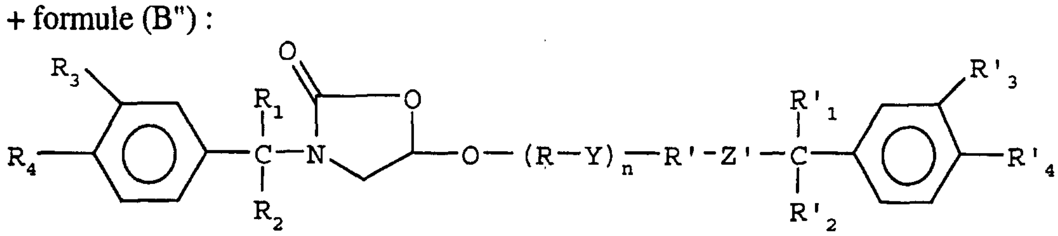

dans laquelle :

- $R_1$, $R_2$, $R_3$, $R_4$, $R'_1$, $R'_2$, $R'_3$, $R'_4$, R, R' et Y sont tels que définis ci-dessus en référence à la formule (B);
- Z' est une fonction carbamate (-O-CO-NH-) ou Z' est une fonction thiocarbamate (-S-CO-NH-); avantageusement, Z' est une fonction carbamate ;
- n est un entier défini de tel sorte que le nombre total d'atomes de carbone, contenus dans la chaîne longue de motif $(R\text{-}Y)_n\text{-}R'$, soit au moins égal à 22 et d'une façon avantageuse soit compris entré 22 et 104 inclus.

7. Matrice (co)polymère selon la revendication 6, **caractérisée en ce que** ledit copolymère est obtenu par polymérisation, par voie radicalaire, d'une composition telle que définie au point d); le(s) monomère(s) difonctionnel(s) de type (a) répondant à la formule (A) dans laquelle :

   - $R_1$ et $R'_1$, identiques, sont un groupe méthyle ;

- R et R', identiques, sont l'hydrogène ou un groupe méthyle ;
- m et n sont indépendamment 1 ou 2 ;
- p et q sont identiques et égaux à 0 ;

et avantageusement R et R' représentent l'hydrogène et m = n = 2 ;
et/ou le(s) oligomère(s) difonctionnel(s) alcénique(s) symétrique(s) à chaîne longue répondant à la formule (B) et/ou à la formule (B') et/ou à la formule (B"), dans lesquelles $R_1$, $R'_1$, $R_2$, $R'_2$ sont identiques et représentent un groupe méthyle, $R_3$ et $R'_3$, identiques, représentent un radical isopropényle et $R_4$ et $R'_4$, identiques, sont l'hydrogène.

**8.** Matrice (co)polymère selon l'une des revendications 6 ou 7, **caractérisée en ce que** ledit copolymère est obtenu par polymérisation, par voie radicalaire, d'une composition telle que définie au point d) de la revendication 6; le(s) oligomère(s) difonctionnel(s) alcénique(s) répondant à la formule (B) et/ou à la formule (B') et/ou à la formule (B"), formules (B), (B'), (B") dans lesquelles la chaîne longue polyoxyalkylène et/ou polymercaptoalkylène, représentée par le motif $(R-Y)_n-R'$ dans le cas d'oligomère(s) de formule (B) ou de formule (B"), ou par le motif $(R-Y)_n$ dans le cas d'oligomère(s) de formule (B'), présente une masse moléculaire comprise entre 500 $g.mol^{-1}$ et 2 000 $g.mol^{-1}$; et avantageusement la masse moléculaire de ladite chaîne longue est au moins de 600 $g.mol^{-1}$ et inférieure à 900 $g.mol^{-1}$.

**9.** Matrice (co)polymère selon l'une quelconque des revendications 6 à 8, **caractérisée en ce que** ledit copolymère est obtenu par polymérisation, par voie radicalaire, d'une composition telle que définie au point d) de la revendication 6; le(s) oligomère(s) difonctionnel(s) de type b) répondant à la formule (B):
dans laquelle :

- Z et Z' sont des fonctions urées (-NH-CO-NH-);
- $R_1$, $R_2$, $R_3$, $R_4$, $R'_1$, $R'_2$, $R'_3$ et $R'_4$ sont tels que définis à la revendication 7 ;
- R' représente un groupe éthylène ou propylène;
- n est un entier égal à 13 ou 19 définissant un nombre total d'atomes de carbone, entre Z et Z', égal à 28 ou 40 quand $(R-Y)_n$ est une chaîne polyoxyéthylène; ou n est un entier égal à 10 ou 14 définissant un nombre total d'atomes de carbone, entre Z et Z', égal à 33 ou 45 quand $(R-Y)_n$ est une chaîne polyoxypropylène; ou n est un entier compris entre les valeurs limites inférieures (n est entre 10 et 13 inclus) et supérieures (n est entre 14 et 19 inclus) telles que définies ci-dessus, quand $(R-Y)_n$ est une chaîne mixte polyoxyéthylène / polyoxypropylène,

ou dans laquelle :

- Z et Z' sont des fonctions carbamates de formule respective (-NH-CO-O-) et (-O-CO-NH-).
- $R_1$, $R_2$, $R_3$, $R_4$, $R'_1$, $R'_2$, $R'_3$ et $R'_4$ sont tels que définis à la revendication 7 ;
- R' représente un groupe éthylène ;
- $(R-Y)_n$ représente une chaîne longue polyoxyéthylène ;
- n est un entier égal à 13 ou 19 définissant un nombre total d'atomes de carbone, contenus dans la chaîne longue située entre les deux motifs Z et Z', égal à 28 ou 40.

**10.** Matrice (co)polymère selon l'une quelconque des revendications 6 à 9, **caractérisée en ce que** ledit copolymère est obtenu par polymérisation, par voie radicalaire, d'une composition telle que définie au point d) de la revendication 6; la quantité de monomère(s) de type (a) étant comprise entre 40 et 99 parties en poids, pour 100 parties en poids du mélange de monomères de type (a) et (b).

**11.** Matrice copolymère selon la revendication 10, **caractérisée en ce qu'**elle contient en outre une quantité efficace d'un amorceur de polymérisation radicalaire.

**12.** Matrice copolymère selon la revendication 11, **caractérisée en ce que** l'amorceur de polymérisation radicalaire est choisi dans le groupe consistant en les amorceurs thermiques, les photoamorceurs et leurs mélanges.

**13.** Produit fini du type vitrage, dispositif optique, article ophtalmique ou solaire consistant avantageusement en une lentille, constitué, en totalité ou en partie, d'une matrice selon l'une quelconque des revendications 5 à 12 et/ou renfermant le composé selon la revendication 4, et avantageusement un mélange des composés selon l'une des revendications 1 ou 2.